# FASCICULE DE BREVET EUROPEEN

(11) **EP 1 539 969 B1**
(45) Date de publication et mention de la délivrance du brevet: **18.07.2012**
(21) Numéro de dépôt: 03760745.4
(22) Date de dépôt: 19.06.2003
(51) Int. Cl.: C12N 15/82, C12N 9/02, C12N 15/62

(54) **PEPTIDE D ADRESSAGE PLASTIDIAL**
PLASTID TRANSIT-PEPTIDE
PLASTIDIAL TARGETING PEPTIDE

(30) Priorité: 21.06.2002 FR 0207729
(43) Date de publication de la demande: 15.06.2005
(73) Titulaire: Genoplante-Valor, 91025 Evry Cedex (FR)
(72) Inventeur: MIRAS, Stéphane, F-38190 Lancey (FR); SALVI, Daniel, F-38210 Tullins (FR); ROLLAND, Norbert, F-38120 Saint-Egrève (FR); JOYARD, Jacques, F-38240 Meylan (FR); FERRO, Myriam, F-38600 Fontaine (FR); GARIN, Jérome, F-38700 Corenc (FR); GRUNWALD, Didier, F-38120 Saint-Egrève (FR)
(74) Mandataire: Vialle-Presles, Marie José
(86) Numéro de dépôt international: PCT/FR2003/001877
(87) Numéro de publication internationale: WO 2004/001050

(56) Documents cités:
- WO-A-02/10210
- WO-A-88/02402
- MIRAS STEPHANE ET AL: "Non-canonical transit peptide for import into the chloroplast." JOURNAL OF BIOLOGICAL CHEMISTRY, vol. 277, no. 49, 6 décembre 2002 (2002-12-06), pages 47770-47778, XP002233838 ISSN: 0021-9258
- SEIGNEURIN-BERNY DAPHNE ET AL: "Differential extraction of hydrophobic proteins from chloroplast envelope membranes: A subcellular-specific proteomic approach to identify rare intrinsic membrane proteins." PLANT JOURNAL, vol. 19, no. 2, juillet 1999 (1999-07), pages 217-228, XP002233839 ISSN: 0960-7412 cité dans la demande
- DATABASE SWALL [en ligne] 1 mai 2000 (2000-05-01) EVAN, M. ET AL.: "Putative zinc-binding dehydrogenase from Arabidopsis" retrieved from EBI, accession no. Q9SV68 Database accession no. Q9SV68 XP002233840
- DATABASE WPI Section Ch, Week 200046 Derwent Publications Ltd., London, GB; Class C06, AN 2000-507395 XP002233841 & EP 1 033 405 A 6 septembre 2000 (2000-09-06)

### SEQUENCE LISTING

<110> GENOPLANTE-VALOR
   MIRAS, Stéphane
   SALVI, Daniel
   ROLLAND, Norbert
   JOYARD, Jacques
   FERRO, Myriam
   GARIN, Jérome
   GRUNWALD, Didier
<120> PEPTIDE D'ADRESSAGE PLASTIDIAL
<130> MJPbv1516/7
<150> FR 02 07729
   <151> 2002-06-21
<160> 18
<170> PatentIn version 3.1
<210> 1
   <211> 329
   <212> PRT
   <213> Arabidopsis thaliana
<400> 1
   Met Ala Gly Lys Leu Met His Ala Leu Gln Tyr Asn Ser Tyr Gly Gly
   1               5                   10                  15
   Gly Ala Ala Gly Leu Glu His Val Gln Val Pro Val Pro Thr Pro Lys
               20                  25                  30
   Ser Asn Glu Val Cys Leu Lys Leu Glu Ala Thr Ser Leu Asn Pro Val
           35                  40                  45
   Asp Trp Lys Ile Gln Lys Gly Met Ile Arg Pro Phe Leu Pro Arg Lys
       50                  55                  60
   Phe Pro Cys Ile Pro Ala Thr Asp Val Ala Gly Glu Val Val Glu Val
   65                  70                  75                  80
   Gly Ser Gly Val Lys Asn Phe Lys Ala Gly Asp Lys Val Val Ala Val
                   85                  90                  95
   Leu Ser His Leu Gly Gly Gly Gly Leu Ala Glu Phe Ala Val Ala Thr
               100                 105                 110
   Glu Lys Leu Thr Val Lys Arg Pro Gln Glu Val Gly Ala Ala Glu Ala
           115                 120                 125
   Ala Ala Leu Pro Val Ala Gly Leu Thr Ala Leu Gln Ala Leu Thr Asn
       130                 135                 140
   Pro Ala Gly Leu Lys Leu Asp Gly Thr Gly Lys Lys Ala Asn Ile Leu
   145                 150                 155                 160
   Val Thr Ala Ala Ser Gly Gly Val Gly His Tyr Ala Val Gln Leu Ala
                   165                 170                 175
   Lys Leu Ala Asn Ala His Val Thr Ala Thr Cys Gly Ala Arg Asn Ile
               180                 185                 190
   Glu Phe Val Lys Ser Leu Gly Ala Asp Glu Val Leu Asp Tyr Lys Thr
           195                 200                 205
   Pro Glu Gly Ala Ala Leu Lys Ser Pro Ser Gly Lys Lys Tyr Asp Ala
       210                 215                 220
   Val Val His Cys Ala Asn Gly Ile Pro Phe Ser Val Phe Glu Pro Asn
   225                 230                 235                 240
   Leu Ser Glu Asn Gly Lys Val Ile Asp Ile Thr Pro Gly Pro Asn Ala
                   245                 250                 255
   Met Trp Thr Tyr Ala Val Lys Lys Ile Thr Met Ser Lys Lys Gln Leu
               260                 265                 270
   Val Pro Leu Leu Leu Ile Pro Lys Ala Glu Asn Leu Glu Phe Met Val
           275                 280                 285
   Asn Leu Val Lys Glu Gly Lys Val Lys Thr Val Ile Asp Ser Lys His
       290                 295                 300
   Pro Leu Ser Lys Ala Glu Asp Ala Trp Ala Lys Ser Ile Asp Gly His
   305                 310                 315                 320
   Ala Thr Gly Lys Ile Ile Val Glu Pro
                   325
<210> 2
   <211> 1228
   <212> DNA
   <213> Spinacia oleracea
<400> 2
   ccatcctaat acgactcact atagggctcg agcggccgcc cgggcaggtc aaactgtggt     60
   aagataatac agtaccatta ccatctgacg cgcaaatggc tgctaagcta atgcatgcga    120
   ttcaatattc tggctatggt ggtggaactg atgctttaaa gcatgttgaa gttgctgttc    180
   ctgatccaaa gtctgatgag ttattgctta aaattgaggc tgcaactttg aacccaattg    240
   attggaagat tcagaagggt gtacttcgtc ccctcttacc ccgcaagttc cctactatac    300
   ctggaactga tgttgctggg gaggtagtcc aggctggatc tgctgtaaat aggtttaaaa    360
   ctggtgacaa agtcgtggcc gtgcttagtc atgctactgg gggtgcacta gctgaatatg    420
   ccgtggcgaa ggagaacctg acagttgcta gaccaccaga agtatcagca gcagaaggtg    480
   ctgccttacc tgttgctgcc ctcacggctc accaagctct cacccagttt gccaacatca    540
   agctcgatgg aagtggtgaa aggaagaaca tattgatcac ggctgcatca gggggtgtgg    600
   gccactatgc ggtccagctg gcaaagctcg ggaacacgca tgtaacagca acatgtggag    660
   cccgcaacct agatttcgtg aaaggcttgg gtgccgatga ggttcttgac tacaaaacac    720
   ctgaaggggc gtccttgaca agcccgtcag gaaagaaata tgactacgta gtccacggtg    780
   caagcggaat cccttggtcc acctttgagc ccaatttgag tgaagcaggt aaggtaatag    840
   atttgactcc tggcccaact gcaatgatga catttgcttg gaaaaagcta acattctcca    900
   aaaagcagct ggtgcctctg cttttgatac caaagatccc caactttgaa tatgttgtga    960
   atttggtaaa ggaaaagaag cttaaaacag tcatagactc taaacatccc ttgagtaaag   1020
   gtgaagatgc ttggagtagg ataatgggtg gtcatgctac agggaagatt ataatcgagc   1080
   cttgaataga aaatattgat gcagacccgc tatatattgc ttgaaggtta caaactttta   1140
   agtttatagt acttgagtta tactttccta gttgtaaaca ttcaagtatt tcataatgtt   1200
   atactttcct agtttcctcc aaaaaaaa                                      1228
<210> 3
   <211> 329
   <212> PRT
   <213> Spinacia oleracea
<400> 3
   Met Ala Ala Lys Leu Met His Ala Ile Gln Tyr Ser Gly Tyr Gly Gly
   1               5                   10                  15
   Gly Thr Asp Ala Leu Lys His Val Glu Val Ala Val Pro Asp Pro Lys
               20                  25                  30
   Ser Asp Glu Leu Leu Leu Lys Ile Glu Ala Ala Thr Leu Asn Pro Ile
           35                  40                  45
   Asp Trp Lys Ile Gln Lys Gly Val Leu Arg Pro Leu Leu Pro Arg Lys
       50                  55                  60
   Phe Pro Thr Ile Pro Gly Thr Asp Val Ala Gly Glu Val Val Gln Ala
   65                  70                  75                  80
   Gly Ser Ala Val Asn Arg Phe Lys Thr Gly Asp Lys Val Val Ala Val
                   85                  90                  95
   Leu Ser His Ala Thr Gly Gly Ala Leu Ala Glu Tyr Ala Val Ala Lys
               100                 105                 110
   Glu Asn Leu Thr Val Ala Arg Pro Pro Glu Val Ser Ala Ala Glu Gly
           115                 120                 125
   Ala Ala Leu Pro Val Ala Ala Leu Thr Ala His Gln Ala Leu Thr Gln
       130                 135                 140
   Phe Ala Asn Ile Lys Leu Asp Gly Ser Gly Glu Arg Lys Asn Ile Leu
   145                 150                 155                 160
   Ile Thr Ala Ala Ser Gly Gly Val Gly His Tyr Ala Val Gln Leu Ala
                   165                 170                 175
   Lys Leu Gly Asn Thr His Val Thr Ala Thr Cys Gly Ala Arg Asn Leu
               180                 185                 190
   Asp Phe Val Lys Gly Leu Gly Ala Asp Glu Val Leu Asp Tyr Lys Thr
           195                 200                 205
   Pro Glu Gly Ala Ser Leu Thr Ser Pro Ser Gly Lys Lys Tyr Asp Tyr
       210                 215                 220
   Val Val His Gly Ala Ser Gly Ile Pro Trp Ser Thr Phe Glu Pro Asn
   225                 230                 235                 240
   Leu Ser Glu Ala Gly Lys Val Ile Asp Leu Thr Pro Gly Pro Thr Ala
                   245                 250                 255
   Met Met Thr Phe Ala Trp Lys Lys Leu Thr Phe Ser Lys Lys Gln Leu
               260                 265                 270
   Val Pro Leu Leu Leu Ile Pro Lys Ile Pro Asn Phe Glu Tyr Val Val
           275                 280                 285
   Asn Leu Val Lys Glu Lys Lys Leu Lys Thr Val Ile Asp Ser Lys His
       290                 295                 300
   Pro Leu Ser Lys Gly Glu Asp Ala Trp Ser Arg Ile Met Gly Gly His
   305                 310                 315                 320
   Ala Thr Gly Lys Ile Ile Ile Glu Pro
                   325
<210> 4
   <211> 41
   <212> PRT
   <213> Arabidopsis thaliana
<400> 4
   Phe Leu Pro Arg Lys Phe Pro Cys Ile Pro Ala Thr Asp Val Ala Gly
   1               5                   10                  15
   Glu Val Val Glu Val Gly Ser Gly Val Lys Asn Phe Lys Ala Gly Asp
               20                  25                  30
   Lys Val Val Ala Val Leu Ser His Leu
           35                  40
<210> 5
   <211> 41
   <212> PRT
   <213> Spinacia oleracea
<400> 5
   Leu Leu Pro Arg Lys Phe Pro Thr Ile Pro Gly Thr Asp Val Ala Gly
   1               5                   10                  15
   Glu Val Val Gln Ala Gly Ser Ala Val Asn Arg Phe Lys Thr Gly Asp
               20                  25                  30
   Lys Val Val Ala Val Leu Ser His Ala
           35                  40
<210> 6
   <211> 327
   <212> PRT
   <213> Escherichia coli
<400> 6
   Met Ala Thr Arg Ile Glu Phe His Lys His Gly Gly Pro Glu Val Leu
   1 5 10 15
   Gln Ala Val Glu Phe Thr Pro Ala Asp Pro Ala Glu Asn Glu Ile Gln
   20 25 30
   Val Glu Asn Lys Ala Ile Gly Ile Asn Phe Ile Asp Thr Tyr Ile Arg
   35 40 45
   Ser Gly Leu Tyr Pro Pro Pro Ser Leu Pro Ser Gly Leu Gly Thr Glu
   50 55 60
   Ala Ala Gly Ile Val Ser Lys Val Gly Ser Gly Val Lys His Ile Lys
   65 70 75 80
   Ala Gly Asp Arg Val Val Tyr Ala Gln Ser Ala Leu Gly Ala Tyr Ser
   85 90 95
   Ser Val His Asn Ile Ile Ala Asp Lys Ala Ala Ile Leu Pro Ala Ala
   100 105 110
   Ile Ser Phe Glu Gln Ala Ala Ala Ser Phe Leu Lys Gly Leu Thr Val
   115 120 125
   Tyr Tyr Leu Leu Arg Lys Thr Tyr Glu Ile Lys Pro Asp Glu Gln Phe
   130 135 140
   Leu Phe His Ala Ala Ala Gly Gly Val Gly Leu Ile Ala Cys Gln Trp
   145 150 155 160
   Ala Lys Ala Leu Gly Ala Lys Leu Ile Gly Thr Val Gly Thr Ala Gln
   165 170 175
   Lys Ala Gln Ser Ala Leu Lys Ala Gly Ala Trp Gln Val Ile Asn Tyr
   180 185 190
   Arg Glu Glu Asp Leu Val Glu Arg Leu Lys Glu Ile Thr Gly Gly Lys
   195 200 205
   Lys Val Arg Val Val Tyr Asp Ser Val Gly Arg Asp Thr Trp Glu Arg
   210 215 220
   Ser Leu Asp Cys Leu Gln Arg Arg Gly Leu Met Val Ser Phe Gly Asn
   225 230 235 240
   Ser Ser Gly Ala Val Thr Gly Val Asn Leu Gly Ile Leu Asn Gln Lys
   245 250 255
   Gly Ser Leu Tyr Val Thr Arg Pro Ser Leu Gln Gly Tyr Ile Thr Thr
   260 265 270
   Arg Glu Glu Leu Thr Glu Ala Ser Asn Glu Leu Phe Ser Leu Ile Ala
   275 280 285
   Ser Gly Val Ile Lys Val Asp Val Ala Glu Gln Gln Lys Tyr Pro Leu
   290 295 300
   Lys Asp Ala Gln Arg Ala His Glu Ile Leu Glu Ser Arg Ala Thr Gln
   305 310 315 320
   Gly Ser Ser Leu Leu Ile Pro
   325
<210> 7
   <211> 334
   <212> PRT
   <213> saccharomyces cerevisiae
<400> 7
<210> 8
   <211> 329
   <212> PRT
   <213> Cavia porcellus
<400> 8
   Met Ala Thr Gly Gln Lys Leu Met Arg Ala Ile Arg Val Phe Glu Phe
   1 5 10 15
   Gly Gly Pro Glu Val Leu Lys Val Gln Ser Asp Val Ala Val Pro Ile
   20 25 30
   Pro Lys Asp His Gln Val Leu Ile Lys Val His Ala Cys Gly Ile Asn
   35 40 45
   Pro Val Glu Thr Tyr Ile Arg Ser Gly Thr Tyr Thr Arg Ile Pro Leu
   50 55 60
   Leu Pro Tyr Thr Pro Gly Thr Asp Val Ala Gly Val Val Glu Ser Ile
   65 70 75 80
   Gly Asn Asp Val Ser Ala Phe Lys Lys Gly Asp Arg Val Phe Thr Thr
   85 90 95
   Ser Thr Ile Ser Gly Gly Tyr Ala Glu Tyr Ala Leu Ala Ser Asp His
               100                 105                 110
   Thr Val Tyr Arg Leu Pro Glu Lys Leu Asp Phe Arg Gln Gly Ala Ala
           115                 120                 125
   Ile Gly Ile Pro Tyr Phe Thr Ala Cys Arg Ala Leu Phe His Ser Ala
       130                 135                 140
   Arg Ala Lys Ala Gly Glu Ser Val Leu Val His Gly Ala Ser Gly Gly
   145                 150                 155                 160
   Val Gly Leu Ala Ala Cys Gln Ile Ala Arg Ala Tyr Gly Leu Lys Val
                   165                 170                 175
   Leu Gly Thr Ala Gly Thr Glu Glu Gly Gln Lys Val Val Leu Gln Asn
               180                 185                 190
   Gly Ala His Glu Val Phe Asn His Arg Asp Ala His Tyr Ile Asp Glu
           195                 200                 205
   Ile Lys Lys Ser Ile Gly Glu Lys Gly Val Asp Val Ile Ile Glu Met
       210                 215                 220
   Leu Ala Asn Val Asn Leu Ser Asn Asp Leu Lys Leu Leu Ser Cys Gly
   225                 230                 235                 240
   Gly Arg Val Ile Ile Val Gly Cys Arg Gly Ser Ile Glu Ile Asn Pro
                   245                 250                 255
   Arg Asp Thr Met Ala Lys Glu Ser Thr Ile Ser Gly Val Ser Leu Phe
               260                 265                 270
   Ser Ser Thr Lys Glu Glu Phe Gln Gln Phe Ala Ser Thr Ile Gln Ala
           275                 280                 285
   Gly Met Glu Leu Gly Trp Val Lys Pro Val Ile Gly Ser Gln Tyr Pro
       290                 295                 300
   Leu Glu Lys Ala Ser Gln Ala His Glu Asn Ile Ile His Ser Ser Gly
   305                 310                 315                 320
   Thr Val Gly Lys Thr Val Leu Leu Met
                   325
<210> 9
   <211> 331
   <212> PRT
   <213> Mus musculus
<400> 9
   Met Ala Thr Gly Gln Lys Leu Met Arg Ala Ile Arg Val Phe Glu Phe
   1               5                   10                  15
   Gly Gly Pro Glu Val Leu Lys Leu Gln Ser Asp Val Val Val Pro Val
               20                  25                  30
   Pro Gln Ser His Gln Val Leu Ile Lys Val His Ala Cys Gly Val Asn
           35                  40                  45
   Pro Val Glu Thr Tyr Ile Arg Ser Gly Ala Tyr Ser Arg Lys Pro Ala
       50                  55                  60
   Leu Pro Tyr Thr Pro Gly Ser Asp Val Ala Gly Ile Ile Glu Ser Val
   65                  70                  75                  80
   Gly Asp Lys Val Ser Ala Phe Lys Lys Gly Asp Arg Val Phe Cys Tyr
                   85                  90                  95
   Ser Thr Val Ser Gly Gly Tyr Ala Glu Phe Ala Leu Ala Ala Asp Asp
               100                 105                 110
   Thr Ile Tyr Pro Leu Pro Glu Thr Leu Asn Phe Arg Gln Gly Ala Ala
           115                 120                 125
   Leu Gly Ile Pro Tyr Phe Thr Ala Cys Arg Ala Leu Phe His Ser Ala
       130                 135                 140
   Arg Ala Arg Ala Gly Glu Ser Val Leu Val His Gly Ala Ser Gly Gly
   145                 150                 155                 160
   Val Gly Leu Ala Thr Cys Gln Ile Ala Arg Ala His Gly Leu Lys Val
                   165                 170                 175
   Leu Gly Thr Ala Gly Ser Glu Glu Gly Lys Lys Leu Val Leu Gln Asn
               180                 185                 190
   Gly Ala His Glu Val Phe Asn His Lys Glu Ala Asn Tyr Ile Asp Lys
           195                 200                 205
   Ile Lys Met Ser Val Gly Asp Lys Asp Lys Gly Val Asp Val Ile Ile
       210                 215                 220
   Glu Met Leu Ala Asn Glu Asn Leu Ser Asn Asp Leu Lys Leu Leu Ser
   225                 230                 235                 240
   His Gly Gly Arg Val Val Val Val Gly Cys Arg Gly Pro Ile Glu Ile
                   245                 250                 255
   Asn Pro Arg Asp Thr Met Ala Lys Glu Thr Ser Ile Ile Gly Val Ser
               260                 265                 270
   Leu Ser Ser Ser Thr Lys Glu Glu Phe Gln Gln Phe Ala Gly Leu Leu
           275                 280                 285
   Gln Ala Gly Ile Glu Lys Gly Trp Val Lys Pro Val Ile Gly Ser Glu
       290                 295                 300
   Tyr Pro Leu Glu Lys Ala Ala Gln Ala His Glu Asp Ile Ile His Gly
   305                 310                 315                 320
   Ser Gly Lys Thr Gly Lys Met Ile Leu Leu Leu
                   325                 330
<210> 10
   <211> 28
   <212> DNA
   <213> Artificial sequence
<220>
   <223> Amorce PCR
<400> 10
   tcacatatgg ctggaaaact caatgcac 28
<210> 11
   <211> 27
   <212> DNA
   <213> Artificial sequence
<220>
   <223> Amorce PCR
<400> 11
   arggatccaa cgctcttatg gctcgac 27
<210> 12
   <211> 30
   <212> DNA
   <213> Artificial sequence
<220>
   <223> Amorce PCR
<400> 12
   cctctcgaga tggctggaaa actcatgcac 30
<210> 13
   <211> 30
   <212> DNA
   <213> Artificial sequence
<220>
   <223> Amorce PCR
<400> 13
   caacccatgg atggctcgac aatgatcttc 30
<210> 14
   <211> 35
   <212> DNA
   <213> Artificial sequence
<220>
   <223> Amorce PCR
<400> 14
   cggttgtcga catgaagagt aatgaggttt gccth 35
<210> 15
   <211> 32
   <212> DNA
   <213> Artificial sequence
<220>
   <223> Amorce PCR
<400> 15
   gaatggtcga catgtttctg ccccgcaagt tc 32
<210> 16
   <211> 32
   <212> DNA
   <213> Artificial sequence
<220>
   <223> Amorce PCR
<400> 16
   ggttgtcgac atgctaggtg gaggtggact tg 32
<210> 17
   <211> 31
   <212> DNA
   <213> Artificial sequence
<220>
   <223> Amorce PCR
<400> 17
   cctctcgaga tggctggaaa aactcatgca c 31
<210> 18
   <211> 30
   <212> DNA
   <213> Artificial sequence
<220>
   <223> Amorce PCR
<400> 18
   acccatggct agatggctaa gaaccgctac 30

## Description

La présente invention est relative à la production de protéines d'intérêt dans des végétaux, et notamment à leur adressage vers le compartiment plastidial.

Les plastes sont des organites intracellulaires des végétaux chlorophylliens (algues, mousses, et végétaux supérieurs). On distingue plusieurs types principaux de plastes, selon leur teneur en pigments, et la nature de ceux-ci : les amyloplastes, riches en amidon, les chloroplastes dans lesquels les pigments principaux sont les chlorophylles, et les chromoplastes dont les pigments principaux sont des caroténoïdes. Ces trois catégories de plastes qui dérivent de précurseurs communs, les proplastes, ont une structure de base commune constituée par une double membrane emprisonnant le stroma plastidial. Dans les chloroplastes, il existe un troisième système membranaire formant à l'intérieur du stroma des saccules dénommées thylakoïdes.

Outre leur rôle primordial dans la photosynthèse, les chloroplastes sont également impliqués dans des réactions d'oxydoréduction, par exemple la réduction des nitrites en ammonium. Les plastes jouent également un rôle essentiel dans la biosynthèse et/ou le stockage de nombreuses molécules, parmi lesquelles on citera l'amidon, les lipides, les caroténoïdes, la plupart des acides aminés, des hormones végétales (acide abscissique, précurseurs des gibbérellines, du jasmonate, etc.).

Bien que les plastes possèdent leur propre génome codant pour une partie de leurs protéines, une grande partie des enzymes intervenant dans les différentes fonctions plastidiales sont codées par le génome nucléaire et importées dans les plastes.

Cette importation s'effectue par un mécanisme spécifique, qui a plus particulièrement été étudié dans le cas des chloroplastes (pour revue cf. CHEN et SCHNELL, Trends Cell Biol. 9, 222-227, 1999 ; KEEGSTRA et CLINE, The Plant Cell 11, 557-570, 1999 ; SCHLEIFF et SOLL, Planta 211, 449-456, 2000 ; JACKSON-CONSTAN et KEEGSTRA, Plant Physiol. 125, 1567-1676, 2001). Ce mécanisme fait intervenir un système d'import dans chacune des deux membranes plastidiales : dans la membrane externe, le complexe TOC (Translocon at Outer membrane of Chloroplast) qui comprend au moins trois protéines : Toc 86, 75 et 34 (KESSLER et al., Science 266, 1035-1039, 1994 ; PERRY et KEEGSTRA, Plant Cell 6, 93-105, 1994) ; dans la membrane interne, le complexe TIC (Translocon at Inner membrane of Chloroplast) qui comprend au moins quatre protéines : Tic 110, 55, 22 et 20 (KESSLER et BLOBEL, Proc. Natl. Acad. Sci. 93, 7684-7689, 1996 ; LÜBECK et al., EMBO J. 15, 4230-4238, 1996 ; CALIEBE et al., EMBO J. 16, 7342-7350, 1997 ; KOURANOV et al., J. Cell Biol., 143, 991-1002, 1998), ainsi qu'une protéine chaperonne dans le stroma : ClpC (AKITA et al., J. Cell Biol. 136, 983-994, 1997 ; NIELSEN et al., EMBO J. 16, 935-946, 1997).

Un élément majeur de ce mécanisme est Toc75, qui est la protéine la plus abondante dans la membrane externe, et forme le pore central du canal de translocation situé dans cette membrane (SCHNELL et al., Science 266, 1007-1012, 1994 ; TRANEL et al., EMBO J. 14, 2436-2446, 1995). Toc75 interagit spécifiquement avec une séquence particulière, dénommée « peptide d'adressage » ou « peptide de transit », localisée à l'extrémité N-terminale des protéines importées dans les plastes (MA et al., J. Cell Biol. 134, 315-327, 1996).

De nombreux peptides d'adressage ont été identifiés dans les précurseurs des protéines adressées vers l'espace intermembranaire, la membrane interne, le stroma, et dans le cas des chloroplastes, vers la membrane du thylakoïde.

Parmi les protéines connues pour posséder un peptide d'adressage intraplastidial clivable, on citera notamment des protéines adressées à l'espace intermembranaire, (Tic22: KOURANOV *et al*., 1998, précédemment cité; KOURANOV et al., J. Biol. Chem. 274, 25181-25194, 1999), des protéines adressées à la membrane interne (TPT(Triose-Pi/Pi translocator) : BRINK et al., J. Biol. Chem. 270, 20808-20815, 1995), des protéines adressées au stroma (petite sous-unité de la ribulose-1,5-bisphosphate carboxylase (Rubisco) : DE CASTRO SILVA FILHO et al., Plant Mol. Biol. 30, 769-780, 1996; *anhydrase carbonique*), des protéines adressées à la membrane du thylakoïde (LHCP(light harvesting complex) : LAMPPA et al., J. Biol. Chem. 263, 14996-14999, 1988; *Cfo-II: ATPase subunit)* et à la lumière du thylakoïde (OEE1 (Oxygen Evolving Element 1) : KO et CASHMORE, EMBO J. 8, 3187-3194, 1989).

Ces peptides d'adressage comprennent généralement entre 40 et 100 acides aminés, et possèdent pour la plupart d'entre eux, des caractéristiques communes : ils sont quasiment dépourvus d'acides aminés chargés négativement, tels que l'acide aspartique, l'acide glutamique, l'asparagine ou la glutamine ; leur région N-terminale est dépourvue d'acides aminés chargés, et d'acides aminés tels que la glycine ou la proline ; leur région centrale contient une proportion très élevée d'acides aminés basiques ou hydroxylés, tels que la sérine et la thréonine ; leur région C-terminale est riche en arginine et a la capacité de former une structure secondaire amphipathique, en feuillet bêta.

Dans le cas des protéines adressées vers la lumière du thylakoïde, le peptide d'adressage est bipartite et comprend des informations additionnelles pour traverser la membrane du thylakoïde (DE BOER et WEISBEEK, Biochim. Biophys. Acta. 1071, 221-253, 1991). Dans certains cas, ce peptide d'adressage bipartite peut aussi être retrouvé chez des protéines adressées vers la membrane du thylakoïde (KARNAUCHOV et al., J. Biol. Chem. 269, 32871-32878, 1994).

Dans tous les cas le peptide d'adressage est clivé après l'importation. Ce clivage est effectué par des protéases spécifiques; une protéase localisée dans le stroma (VANDERVERE et al., Proc. Natl. Acad. Sci. 92, 7177-7181, 1995), et une protéase localisée dans la lumière du thylakoïde (CHAAL et al., J. Biol. Chem. 273, 689-692, 1998) ont été décrites.

Les protéines adressées à la membrane externe ne comportent généralement pas de peptide signal clivable ; l'information d'adressage est contenue dans la protéine mature (CLINE et HENRY, Annu. Rev. Cell Dev. Biol., 12, 1-26, 1996) ; après leur synthèse dans le cytosol, ces protéine sont directement incorporées dans la membrane (VAN'T HOF et al, FEBS lett. 291, 350-354, 1991 et J. Biol. Chem. 268, 4037-4042, 1993 ; PINADUWAGE et BRUCE, J. Biol. Chem. 271, 32907-32915, 1996) par l'intermédiaire d'interactions, dont la nature demeure mal connue, avec la bicouche lipidique. La seule exception connue à ce jour concerne la protéine Toc75 ou (OEP75) dont l'adressage à la membrane externe nécessite la présence d'un peptide d'adressage N-terminal bipartite clivable (TRANEL et al., 1995, précédemment cité; TRANEL and KEEGSTRA, Plant Cell 8, 2093-2104, 1996).

Il est connu que l'utilisation des peptides d'adressage aux plastes est nécessaire pour introduire dans ceux-ci des protéines d'intérêt permettant d'agir sur diverses fonctions plastidiales notamment dans le but d'améliorer les caractéristiques de plantes d'intérêt agronomique, par exemple la biosynthèse des lipides, de l'amidon, des vitamines, des hormones ou des protéines par lesdites plantes, ou leur résistance aux maladies aux insectes ou aux herbicides. Par exemple, la Demande EP 189707, ainsi que la Demande PCT WO 88/02402 proposent l'utilisation de peptides d'adressage clivables issus de précurseurs de protéines chloroplastiques, et en particulier du peptide d'adressage de la petite sous-unité de la ribulose-1,5-bisphosphate carboxylase, pour importer une protéine d'intérêt dans des chloroplastes ; la Demande PCT WO 00/12732 propose l'utilisation de peptides d'adressage de différentes protéines plastidiales pour importer des protéines d'intérêt dans des plastes.

Les fonctions plastidiales pouvant être modifiées de la sorte, et les caractéristiques conférées par ces modifications sont très diverses.

A des fins d'illustration non-limitative, on peut citer :
- l'augmentation de la résistance aux herbicides, par expression du précurseur de l'acétolactate synthétase (ALS), (LEE et al. EMBO J., 7, 1241-1248, 1988) d'acétolactate synthétase mutée (PRESTON et POWLES, Heredity 88, 8-13, 2002) ; CHONG et CHOI, Biochem. Biophys. Res. Commun. 279, 462-467, 2000), ou de la 3-énolpyruvylshikimate-5-phosphate synthétase (EPSP synthétase)(KLEE et al., Mol. Gen. Genet., 210, 437-442, 1987)
- l'augmentation de la résistance à différents stress, par expression de la zéaxanthine époxidase, (SEO et al., Trends Plant Sci., 7, 41-48, 2002), de la choline monooxygénase (SHEN et al., Sheng Wu Gong Cheng Xue Bao, 17, 1-6, 2001), du produit du gène ERD1_ARATH (KIYOSUE et al., Biochem. Biophys. Res. Commun., 15, 196, 1214-1220, 1993), de la ferrochélatase (CHOW et al., J. Biol. Chem., 31, 272, 27565-27571, 1997), de l'omega-3 acide gras désaturase (IBA et al., Tanpakushitsu Kakusan Koso, 39, 2803-2813, 1994; MURAKAMI et al., Science 21, 287(5452), 476-479, 2000), de la glutamine synthétase (FUENTES et al., J. Exp. Bot., 52, 1071-1081, 2001)
- la modification du métabolisme des plastes, pour augmenter la capture d'énergie lumineuse, (GAUBIER et al., Mol. Gen. Genet., 1, 249, 58-64, 1995), les capacités de photosynthèse et de croissance, (MIYAGAWA et al., Nature Biotech., 19, 965-969, 2001), la teneur en caroténoïdes (HUGUENEY et al., Eur. J. Biochem., 1, 209, 399-407, 1992 ; MANN et al., Nature Biotech., 18, 888-892, 2000), ou la teneur en différentes substances d'intérêt, telles que l'amidon (Demande PCT WO 00/11144), des acides aminés essentiels (MUEHLBAUER et al., Plant Physiol., 106, 1303-1312, 1994), la provitamine A (RÖMER et al., Nature Biotech., 18, 666-669, 2000), des hormones (JOYARD et al., Plant Physiol. 118, 715-723, 1998), etc.
- la surexpression et l'adressage chloroplastique de protéines utilisables à des fins de bioremédiation (détoxification ou dépollution des sols contaminés) telles que la ferritine, (LOBREAUX et al., Biochem. J., 15, 288(Pt 3), 931-939, 1992), les protéines de la famille des phytochélatines (CAZALE et CLEMENS, FEBS 507, 215-219, 2001 ; TSUJI et al., BBRC 293, 653-659, 2002), etc.

Tous les peptides d'adressage intraplastidial connus dans l'art antérieur permettent d'importer une protéine dans les plastes par l'intermédiaire des systèmes membranaires d'import TOC et TIC, comme indiqué ci-dessus. Il a été constaté que l'utilisation de ces peptides pour l'adressage de protéines d'intérêt dans le chloroplaste pouvait, notamment dans les cas où la construction peptide d'adressage/protéine d'intérêt est placée sous contrôle d'un promoteur fort tel que le promoteur 35S, présenter l'inconvénient de saturer ces systèmes d'import, en entrant en compétition avec les protéines adressées naturellement au chloroplaste. Il en résulte des « fuites » se traduisant au bout de quelques jours par la présence de la protéine d'intérêt dans d'autres compartiments subcellulaires comme le cytoplasme.

Il serait souhaitable de disposer de peptides d'adressage intraplastidial, qui ne dépendraient pas du système d'import TOC/TIC, et permettraient donc d'éviter les inconvénients mentionnés ci-dessus.

Lors de travaux précédents visant à identifier, par une approche protéomique, des protéines de préparations de membranes de chloroplastes d'épinard, les Inventeurs ont identifié, entre autres, des peptides possédant une similitude de séquence importante avec une protéine putative de 41 kDa d'*Arabidopsis* (numéro d'accès TrEMBL Q9SV68) (SEIGNEURIN-BERNY et al., Plant. J. 19, 217-228, 1999; FERRO et al., Electrophoresis 21, 3517-3526, 2000). Des séquences nucléotidiques codant pour cette protéine putative *d'Arabidopsis* sont par ailleurs mentionnées dans les Demandes PCT WO 02/10210 et EP 1033405.

En poursuivant leurs travaux afin de mieux caractériser la protéine d'*Arabidopsis,* et son homologue chez l'épinard, les Inventeurs ont constaté que, de manière surprenante, bien qu'il s'agisse de protéines synthétisées dans le cytoplasme et importées au niveau de la membrane interne du chloroplaste, leur importation s'effectuait sans clivage d'un peptide d'adressage ; en outre, l'analyse de séquences de ces protéines ne fait apparaître aucune séquence possédant les caractéristiques des peptides d'adressage plastidial connus.

Les protéines de la famille représentée par la protéine de 41 kDa d'*Arabidopsis,* et la protéine homologue d'épinard sont désignées ci-après sous la dénomination IE41. (IE pour Inner Envelope selon la nomenclature classiquement utilisée pour ce système membranaire).

La séquence de la protéine IE41 d'*Arabidopsis* est représentée dans la liste de séquences en annexe sous le numéro SEQ ID NO: 1 ; la séquence de l'ADNc codant pour la protéine IE41 d'épinard est représentée dans la liste de séquences en annexe sous le numéro SEQ ID NO:2, et la séquence polypeptidique correspondante est représentée dans la liste de séquences en annexe sous le numéro SEQ ID NO:3.

Les Inventeurs ont recherché quelles étaient les régions des protéines IE41 impliquées dans leur adressage plastidial, et ont identifié une région de 41 acides aminés (résidus 60 à 100) essentielle à cet adressage.

La séquence de cette région est représentée dans la liste de séquences en annexe sous le numéro SEQ ID NO: 4 pour la protéine IE41 d'*Arabidopsis,* et sous le numéro SEQ ID NO: 5 pour la protéine IE41 d'épinard.

Ils ont en outre constaté que lorsque des fragments d'IE41 contenant cette région étaient fusionnés à l'extrémité N-terminale d'une protéine hétérologue, la protéine recombinante résultant de cette fusion était adressée aux chloroplastes de manière similaire à la protéine IE41 entière.

La présente invention a pour objet un polypeptide d'adressage intraplastidial caractérisé en ce qu'il est constitué par :
- un domaine A constitué par un polypeptide présentant au moins 60%, de préférence au moins 70%, avantageusement au moins 80%, et de manière tout à fait préférée au moins 90% d'identité, ou au moins 65%, de préférence au moins 75%, avantageusement au moins 85%, et de manière tout à fait préférée au moins 95% de similarité, avec l'un des polypeptides SEQ ID NO: 4 ou SEQ ID NO: 5 ;
   et au moins un domaine choisi parmi :
- un domaine B situé à l'extrémité N-terminale du domaine A, et constitué par un fragment de l'un des polypeptides SEQ ID NO: 1 ou SEQ ID NO: 3 comprenant au moins les acides aminés 49 à 59, de préférence au moins les acides aminés 39 à 59, avantageusement au moins les acides aminés 29 à 59, de manière tout à fait préférée au moins les acides aminés 19 à 59, et de manière particulièrement avantageuse au moins les acides aminés 9 à 59 dudit polypeptide, ou bien par un polypeptide présentant au moins 60%, de préférence au moins 70%, avantageusement au moins 80%, et de manière tout à fait préférée au moins 90% d'identité, ou au moins 65%, de préférence au moins 75%, avantageusement au moins 85%, et de manière tout à fait préférée au moins 95% de similarité, avec ledit fragment ;
- un domaine C situé à l'extrémité C-terminale du domaine A, et constitué par un fragment de l'un des polypeptides SEQ ID NO: 1 ou SEQ ID NO: 3 comprenant au moins les acides aminés 101 à 111, de préférence au moins les acides aminés 101 à 121, avantageusement au moins les acides aminés 101 à 131, de manière tout à fait préférée au moins les acides aminés 101 à 141, et au plus les acides aminés 101 à 151 dudit polypeptide, ou bien par un polypeptide présentant au moins 60%, de préférence au moins 70%, avantageusement au moins 80%, et de manière tout à fait préférée au moins 90% d'identité, ou au moins 65%, de préférence au moins 75%, avantageusement au moins 85%, et de manière tout à fait préférée au moins 95% de similarité, avec ledit fragment.

Les pourcentages d'identité ou de similarité mentionnés ici sont déterminés à l'aide du logiciel BLASTp (ALTSCHUL et al., Nucleic Acids Res. 25, 3389-3402, 1997), en utilisant les paramètres par défaut.

Les domaines A, B, et/ou C définis ci-dessus peuvent provenir d'une même protéine IE41 ; ils peuvent également provenir de protéines IE41 d'origines différentes.

La présente invention a également pour objet tout polypeptide chimérique résultant de la fusion d'un polypeptide d'adressage intraplastidial conforme à l'invention avec un polypeptide hétérologue. Ledit polypeptide hétérologue peut être n'importe quel polypeptide d'intérêt que l'on souhaite introduire dans les plastes. De préférence, le polypeptide d'adressage intraplastidial conforme à l'invention est placé à l'extrémité N-terminale du peptide hétérologue. Il pourrait toutefois être également placé à l'intérieur de celui-ci, ou bien à son extrémité C-terminale.

La présente invention a également pour objet l'utilisation d'un polypeptide d'adressage intra-plastidial conforme à l'invention pour l'importation d'une protéine d'intérêt dans des plastes, et avantageusement, pour l'adressage de ladite protéine à la membrane interne plastidiale.

Selon un mode de mise en oeuvre préféré de la présente invention, ledit polypeptide d'adressage intra-plastidial est utilisé pour l'importation de ladite protéine d'intérêt dans des chloroplastes.

En particulier, la présente invention a pour objet un procédé pour importer une protéine d'intérêt dans des plastes caractérisé en ce qu'il comprend l'expression, dans une cellule végétale contenant lesdits plastes, d'un polypeptide chimérique résultant de la fusion d'un polypeptide d'adressage intraplastidial conforme à l'invention avec un polypeptide hétérologue.

La présente invention a également pour objet :
- tout polynucléotide codant pour un polypeptide d'adressage intraplastidial ou pour un polypeptide chimérique conforme à l'invention ;
- toute cassette d'expression recombinante comprenant un polynucléotide conforme à l'invention placé sous contrôle de séquences appropriées de régulation de la transcription (notamment promoteur et terminateur de transcription).
- tout vecteur recombinant résultant de l'insertion, dans un vecteur-hôte approprié, d'un polynucléotide ou d'une cassette d'expression conforme à l'invention.

La présente invention a aussi pour objet des cellules-hôtes hébergeant un polynucléotide, une cassette d'expression, ou un vecteur recombinant conforme à l'invention.

La présente invention englobe également des plantes transgéniques génétiquement transformées par un polynucléotide ou une cassette d'expression conforme à l'invention, ainsi que les descendants de ces plantes. L'invention comprend également les cellules et tissus végétaux, ainsi que les organes ou parties de plantes, y compris feuilles, tiges, racines, fleurs, fruits, et/ou graines obtenues à partir de ces plantes.

Les techniques classiques de construction de vecteurs recombinants, de transformation de cellules ou d'organismes hôte, et de production de protéines recombinantes, sont utilisables pour la mise en oeuvre de la présente invention.

Le choix du vecteur-hôte, et des séquences de régulation de l'expression seront effectuées notamment en fonction de la méthode de transformation et de la plante hôte choisies, et/ou du type de cellule ou de tissu dans lequel on souhaite obtenir l'expression.

De très nombreux promoteurs utilisables pour l'expression dans des cellules végétales sont connus en eux-mêmes. A titre d'exemples, on peut choisir un promoteur constitutif, tel que le promoteur 35S du CaMV ou ses dérivés, ou le promoteur de l'actine ou de l'ubiquitine, etc. On peut également choisir un promoteur inductible ou bien un promoteur tissu-spécifique, afin de n'effectuer l'adressage plastidial de la protéine d'intérêt qu'à certains stades du développement de la plante, dans certaines conditions environnementales, ou dans certains tissus-cibles.

Par exemple, si l'on souhaite obtenir préférentiellement un adressage de la protéine d'intérêt vers les chloroplastes, on exprimera un polypeptide chimérique conforme à l'invention sous contrôle d'un promoteur spécifique de tissus ou organes riches en plastes. A titre d'exemples, les promoteurs du virus de la Chlorelle régulant l'expression du gène de l'adénine méthyltransférase (MITRA et HIGGINS, Plant Mol. Biol. 26, 85-93, 1994) ou celui du virus de la mosaïque du manioc (VERDAGUER et al., Plant Mol. Biol. 37, 1055-1067, 1998) s'expriment principalement dans les tissus verts. Les éléments régulateurs du promoteur du gène 2A11 de la tomate permettent une expression spécifique dans les fruits (VAN HAAREN et HOUCK, Plant Mol. Biol. 17, 615-630, 1991), etc.

Des méthodes de transformation de cellules végétales ou de plantes entières sont bien connues en elles-mêmes : à titre d'exemples non-limitatifs, on citera la transformation de protoplastes en présence de polyéthylèneglycol, l'électroporation, l'utilisation d'un canon à particules, la micro-injection cytoplasmique ou nucléaire, ou la transformation par l'intermédiaire d'*Agrobacterium.*

La présente invention peut être mise en oeuvre dans les applications usuelles des peptides d'adressage aux plastes, et notamment dans celles mentionnées plus haut, pour agir sur diverses fonctions plastidiales. Il s'agit, en particulier, de la modification de fonctions propres à la membrane interne de l'enveloppe, par exemple, les biosynthèses de pigments, de quinones, d'acides gras, de vitamines et de précurseurs d'hormones végétales, mais aussi, l'import de tous les ions et métabolites vers le plaste.

Les caractéristiques des peptides d'adressage plastidial conformes à l'invention, qui sont très différentes de celles des peptides d'adressage plastidial connus permettent de supposer que les peptides d'adressage conformes à l'invention utilisent un système d'import différent de celui impliquant les protéines TOC et TIC.

De ce fait, les protéines d'intérêt adressées aux plastes à l'aide d'un peptide d'adressage conforme à l'invention n'entreraient pas en compétition avec les protéines naturellement adressées vers le plaste par l'intermédiaire du système TOC et TIC, et ne satureraient pas ce dernier. Ceci permettrait notamment d'éviter les fuites dans les autres compartiments subcellulaires, et de conserver les protéines d'intérêt dans le chloroplaste, même après plusieurs jours d'expression. Ceci permettrait également d'adresser vers les plastes des protéines pour lesquelles l'import classique grâce à une séquence d'adressage clivable en N-terminal et utilisant le système TIC/TOC, ne serait pas fonctionnel.

La présente invention sera mieux comprise à l'aide du complément de description qui va suivre, qui se réfère à des exemples non-limitatifs illustrant l'obtention et la caractérisation de peptides d'adressage plastidial conformes à l'invention et leur mise en oeuvre pour l'importation de protéines hétérologues dans les chloroplastes.

### EXEMPLE 1 : CARACTERISATION ET CLONAGE DE LA PROTEINE IE41 D'ARABIDOPSIS THALIANA

Lors de travaux antérieurs visant à identifier les protéines les plus hydrophobes de préparations de membranes chloroplastiques d'épinard (SEIGNEURIN-BERNY et al., Plant. J., 19, p.217-228, 1999 ; FERRO et al., Electrophoresis, 21, 3517-3526, 2000), plusieurs peptides dérivant d'une protéine de 41 kDa présentant une forte similarité de séquence avec une protéine putative d'*Arabidopsis* (Numéro d'accession TrEMBL Q9SV68) ont été mis en évidence.

Cependant, l'analyse de la séquence primaire de cette protéine de 41 kDa à l'aide du programme TMPred (HOFMANN et STOFFEL, Biol. Chem. Hoppe-Seyler., 347, 166, 1993), n'a pas permis de détecter de segments trans-membranaires pouvant assurer l'ancrage de la protéine dans une bicouche lipidique.

Pour confirmer la localisation de cette protéine dans l'enveloppe du chloroplaste, l'ADNc correspondant a été cloné et la protéine recombinante sur-exprimée chez *E*. *coli* afin d'obtenir des anticorps polyclonaux dirigés contre cette protéine.

### Expression chez E. coli

L'ADNc codant pour la protéine de 41 kDa *d'Arabidopsis* est obtenu par PCR, à partir d'une librairie d'ADNc *d'Arabidopsis,* en utilisant les amorces suivantes :
TCACATATGGCTGGAAAACTCAATGCAC (SEQ ID NO : 10)
   qui permet d'introduire un site de restriction *NdeI* (souligné) à l'extrémité 5' de l'ADNc ;
ATGGATCCAACGCTCTTATGGCTCGAC (SEQ ID NO : 11)
   qui permet d'introduire un site de restriction *BamHI* (souligné) à l'extrémité 3' de l'ADNc.

Le fragment d'amplification est cloné dans le plasmide pBluescript KS⁻. L'insert est ensuite digéré par les enzymes de restriction *NdeI* et *BamHI,* et inséré dans le vecteur d'expression pET-15b (NOVAGEN).

Le vecteur résultant permet l'expression d'une protéine recombinante ayant une extension polyhistidine (His-tag) à son extrémité N-terminale [(His-tag)-P41].

Ce vecteur est utilisé pour transformer des bactéries *E*. *coli,* souche BLR(DE3).

Les bactéries recombinantes sont mises en culture dans 500 ml de milieu LB contenant 100 µg/ml d'ampicilline à 37°C. Quand la densité optique à 600 nm (DO₆₀₀) de la culture d'*E. coli* atteint 0,6, on ajoute de l'IPTG (isopropyl-β-D-galactothiopyranoside) à une concentration finale de 1 mM, pour induire l'expression de la protéine de 41 kDa.

Après 3 heures de culture, les cellules sont centrifugées pendant 2 mn à 13 000 rpm (EPPENDORF 5415D).

Le culot est remis en suspension dans 20 ml de tampon de lyse (50 mM NaH₂PO₄, 300 mM Na Cl, 10 mM imidazole, pH 8) et les bactéries sont lysées par sonication (sonicateur OMRON, type STP.YM.220.VAC, 6×1 mn, 0°C).

Après sonication, l'extrait protéique total est analysé par SDS-PAGE 12%. Les gels sont colorés au bleu de Coomassie (R-250, BIORAD).

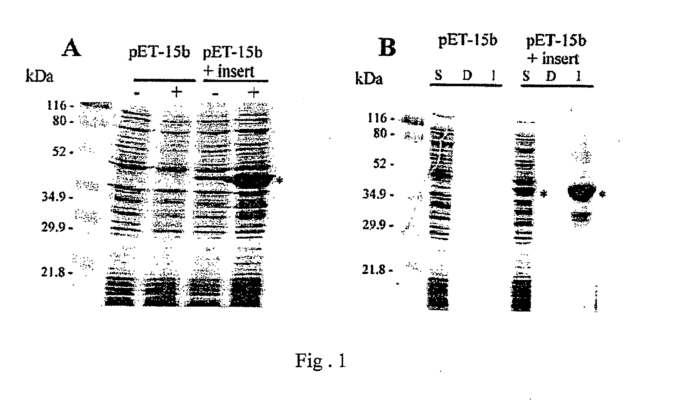

Les résultats sont illustrés par la figure 1A : Légende de la figure 1A :
pET-15b = bactéries transformées par le plasmide non recombinant (contrôle négatif)
pET-15b + insert = bactéries transformées par le plasmide recombinant
- = pas d'induction par IPTG
+ = induction par IPTG
* = bande correspondant à la protéine de 41 kDa recombinante

Après induction par l'IPTG, la protéine recombinante est fortement exprimée dans les bactéries transformées par le plasmide pET-15b comprenant l'insert d'ADNc d'*Arabidopsis.*

### Solubilisation de la protéine recombinante

Le culot bactérien est mis en suspension dans 1 ml de tampon Tris/HCl 20 mM, pH 6,8 puis lysé par sonication (sonicateur OMRON, type STP.YM.220.VAC, 6x1 mn, 0°C).

Après sonication, une première centrifugation (2 mn, 13 000 rpm) de l'extrait protéique total permet d'isoler les protéines solubles du surnageant. Les protéines insolubles du culot sont mises en suspension dans 1 ml d'un tampon contenant du détergent (20 mM Tris/HCl pH 6,8, 0,5% Triton X-100). Une seconde centrifugation permet d'isoler les protéines membranaires solubilisées par le Triton X-100. Les protéines non solubilisées sont remises en suspension dans 20 mM Tris/HCl, pH 6,8 et analysées. Les différentes fractions sont analysées par SDS-PAGE 12%, comme indiqué ci-dessus.

Les résultats sont illustrés par la figure 1B : Légende de la figure 1B :
pET-15b = bactéries transformées par le plasmide non recombinant (contrôle négatif)
pET-15b + insert = bactéries transformées par le plasmide recombinant
S = protéines solubles
D = protéines solubilisées dans le Triton X-100
1 = protéines non solubilisées dans le Triton X-100
* = bande correspondant à la protéine de 41 kDa recombinante

On note que la protéine recombinante (*) se retrouve dans 2 fractions distinctes : une fraction hydrosoluble présente dans le cytosol d'*E. coli* et une fraction insoluble, qui n'est pas solubilisée par le Triton X-100, ce qui indique qu'elle est probablement agrégée sous forme de corps d'inclusion.

### Purification de la protéine recombinante

La fraction soluble de la protéine (His_tag) - P41 recombinante est purifiée par chromatographie d'affinité.

Après centrifugation (10 mn à 6 000 rpm, EPPENDORF 5415D), le surnageant est déposé sur une colonne d'affinité « His-bind resin », (NOVAGEN) de 2,5 ml chargée avec 13 ml de tampon de charge (50 mM NiSO₄) et équilibrée par 5 ml de tampon d'équilibrage (20 mM Tris/HCl pH 7,9, 5 mM imidazole, 0,5 M NaCl).

La colonne est lavée avec 2 volumes contenant 35 mM imidazole, et 2 volumes de tampon de lyse contenant 60 mM imidazole (L2). La protéine est éluée avec 6 volumes de tampon de lyse contenant 250 mM imidazole. Les différentes fractions sont analysées par SDS-PAGE 12% et révélées au bleu de Coomassie.

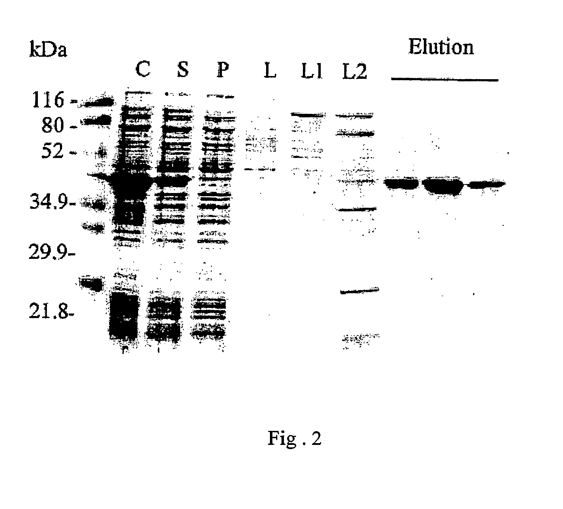

Les résultats sont illustrés par la figure 2. Légende de la figure 2 :
C = culot bactérien dilué dans le tampon de lyse (10 µl)
S = protéines bactériennes solubles (10 µl)
P = protéines non fixées (10 µl)
L, L1, L2 = lavages avec le tampon de lyse (35 et 60 mM imidazole) (15 µl)
Elution = fraction éluée en présence de 250 mM imidazole.

### Production d'anticorps polyclonaux

La protéine recombinante (His_tag)-P41 purifiée, est dessalée (colonne SEPHADEX G25) et stockée à -80°C.

Cette protéine recombinante est utilisée pour immuniser des lapins afin de produire des anticorps polyclonaux dirigés contre la protéine de 41 kDa d'*Arabidopsis.*

### EXEMPLE 2 : LOCALISATION DE LA PROTEINE DE 41 KDA DANS LES CHLOROPLASTES

Lors de la procédure de purification, la protéine de 41 kDa se comporte comme une protéine hydrosoluble et faiblement hydrophobe, ce qui pose la question de son association effective avec l'enveloppe du chloroplaste.

Sa localisation sub-cellulaire a donc été vérifiée par analyse de différentes fractions chloroplastiques.

Des chloroplastes bruts sont obtenus à partir de 3-4 kg de feuilles d'épinard (*Spinacia oleracea L*.) et sont purifiés par centrifugation isopycnique sur gradient de Percoll (DOUCE et JOYARD, Methods in chloroplast Molecular Biology. Edelman, M., Hallick, R. and Chua, N.-H., eds. (Amsterdam : Elsevier Science Publishers BV), 239-256, 1982). A ce stade, des inhibiteurs de protéases (1 mM PMSF, 1 mM benzamidine et 0,5 mM acide amino caproïque) sont ajoutés afin d'empêcher toute dégradation protéique. Les chloroplastes purifiés sont lysés dans un milieu hypotonique, et les membranes de l'enveloppe sont purifiées à partir du lysat par centrifugation sur gradient de sucrose (DOUCE ET JOYARD, 1982, précité). Des sous-fractions d'enveloppe respectivement enrichies en membranes externes et internes sont obtenues selon le protocole décrit par BLOCK et al. (J. Biol. Chem., 258, 13273-13280, 1983).

Toutes les étapes ci-dessus sont réalisées entre 0 et 5°C. Les fractions obtenues sont stockées en azote liquide dans 50 mM MOPS-NaOH, pH 7,8, en présence d'inhibiteurs de protéases (1 mM benzamidine et 0,5 mM acide amino caproïque).

### Analyse des fractions chloroplastiques

Les analyses par SDS-PAGE des chloroplastes totaux, ou de leurs fractions membranes d'enveloppe, stroma, membranes des thylakoïdes ; 15 µg) ainsi que des des sous-fractions d'enveloppe de chloroplastes (15 µg) sont effectuées comme décrit par CHUA (Methods Enzymol., 69, 434-436, 1980). Les gels de résolution et de concentration (12-15% acrylamide), comme le tampon de migration, contiennent 0,1% de SDS. Les peptides sont révélés soit par le bleu de Coomassie (MANIATIS et al., Cold Spring Harbor Laboratory, Cold Spring Harbor, NY, 1982) soit par le nitrate d'argent (MERRIL et al., Anal. Biochem., 110, 201-207, 1981).

Pour les analyses par Western blot, la protéine de 41 kDa est détectée en utilisant les anticorps polyclonaux dirigés contre la protéine recombinante d'*Arabidopsis* produite comme décrit à l'exemple 1, marqués à la phosphatase alcaline.

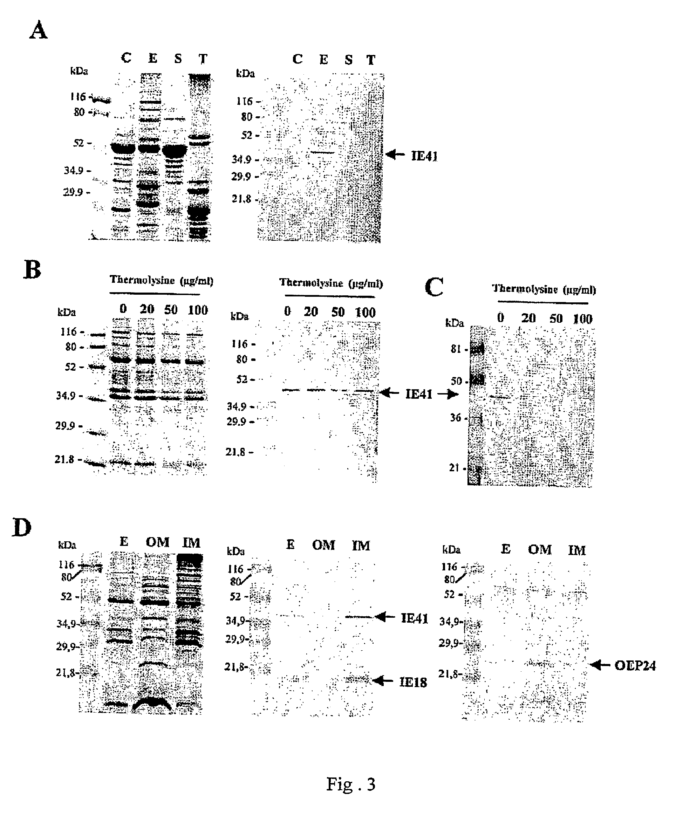

Les résultats sont représentés sur la figure 3A.
Légende de la figure 3A :
C = protéines du chloroplaste
E = protéines des membranes d'enveloppe
S = protéines du stroma
T = protéines des membranes des thylakoïdes

Ces résultats montrent que la protéine de 41 kDa est associée uniquement avec l'enveloppe du chloroplaste et n'est pas détectée dans le stroma ni dans les thylakoïdes.

Les chloroplastes intacts purifiés sont dépourvus de protéines du cytosol qui peuvent contaminer la préparation (DOUCE et JOYARD, 1980, précité). Toutefois, la protéine de 41 kDa pourrait interagir spécifiquement avec la membrane externe de l'enveloppe du chloroplaste et pourrait ainsi être co-purifiée avec les préparations d'enveloppe. Afin d'exclure cette possibilité, 20 µg d'enveloppe issue de chloroplastes intacts sont traités avec la thermolysine de *Bacillus thermoproteolyticus* (0, 20, 50 et 100 µg/ml) afin de digérer les polypeptides localisés sur la face externe de la membrane externe de l'enveloppe (JOYARD et al., J. Biol. Chem., 258, 10000-10006, 1983). A titre de contrôle, des protéines d'enveloppe solubilisées subissent le même traitement protéolytique.

La présence de la protéine de 41 kDa est détectée par Western blot, comme décrit ci-dessus.

Les résultats sont représentés dans les figures 3B et 3C.
Légende des figures 3B et 3C :
0 = absence de thermolysine
20 = thermolysine à 20 µg/ml
50 = thermolysine à 50 µg/ml
100 = thermolysine à 100 µg/ml

La protéine de 41 kDa n'est pas affectée par le traitement par la thermolysine (figure 3B), alors que le même traitement protéolytique réalisé sur des protéines d'enveloppe solubilisées montrent la sensibilité de la protéine de 41 kDa au traitement par la thermolysine (figure 3C). Ce résultat exclut l'hypothèse selon laquelle la protéine de 41 kDa serait localisée sur la face externe de la membrane externe. De fait, la protéine de 41 kDa n'est pas une protéine du cytosol contaminant les préparations d'enveloppe de chloroplaste.

Des sous-fractions d'enveloppe respectivement enrichies en membranes externes et internes sont utilisées pour préciser la sous-localisation de la protéine de 41 kDa au niveau de la membrane de l'enveloppe du chloroplaste. La nature de ces sous-fractions d'enveloppe a été confirmée par l'utilisation des marqueurs IE18 et OEP24, qui sont respectivement des protéines intrinsèques de la membrane interne et externe de l'enveloppe du chloroplaste. Les protéines IE18 et OEP24 sont détectées en utilisant des anticorps polyclonaux dirigés spécifiquement contre chacune de ces protéines.
Les résultats sont représentés dans la figure 3D.
Légende de la figure 3D :
E = protéines des membranes de l'enveloppe
OM = protéines de la membrane externe
IM = protéines de la membrane interne

La protéine de 41 kDa se retrouve, comme la protéine IE18, uniquement dans les préparations d'enveloppes chloroplastiques enrichies en membrane interne.

L'ensemble des résultats représentés dans les figures 3A-3D montre que la protéine de 41 kDa est localisée au niveau de la membrane interne de l'enveloppe du chloroplaste.

D'après la nomenclature classique, cette protéine de l'enveloppe interne, présentant une masse théorique en électrophorèse sur gel de polyacrylamide, de 41 kDa, est dénommée IE41 (pour « Inner Envelope Protein of 41 kDa »).

### Analyse des interactions entre la protéine IE41 et la membrane interne de l'enveloppe du chloroplaste

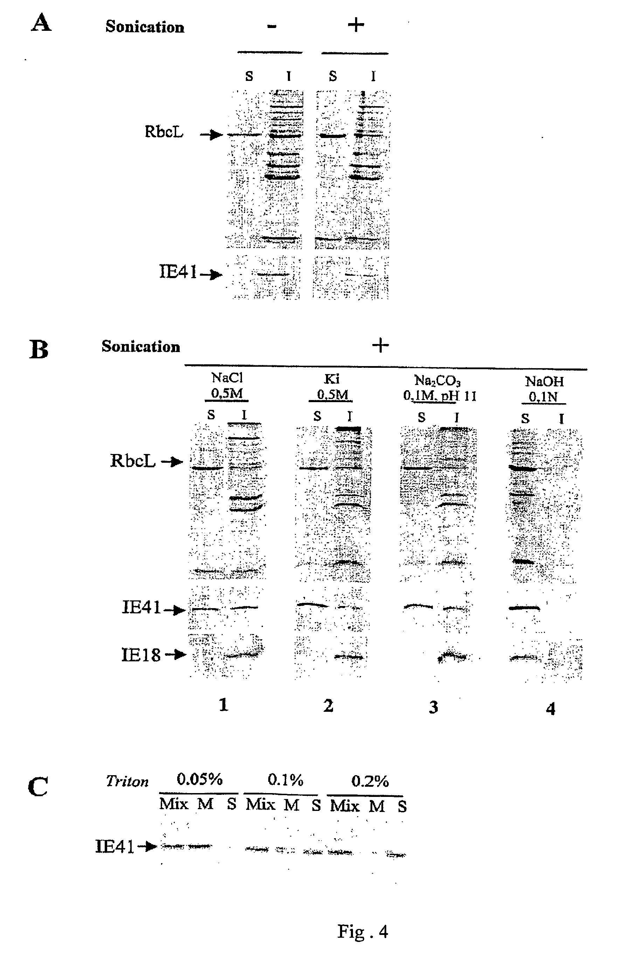

Pour analyser plus précisément le mode d'interaction de la protéine IE41 avec la membrane interne des chloroplastes, différentes hypothèses ont été testées :
1) La protéine IE41 serait une protéine soluble localisée dans l'espace intermembranaire situé entre les membranes externe et interne de l'enveloppe du chloroplaste. Cette protéine serait co-purifiée avec les préparations d'enveloppe, et séquestrée dans les vésicules d'enveloppe. Dans ce cas la sonication des vésicules d'enveloppe permettrait la libération de IE41.
   Pour tester cette première hypothèse, des protéines d'enveloppe (500 µg) sont solubilisées dans 50 mM MOPS, pH 7,8 (500 µl). Les vésicules d'enveloppe sont soniquées pendant 10 sec puis centrifugées (20 mn à 72 000 g, Beckman L2 65B, rotor SW28) afin de séparer les protéines solubles et membranaires. Chaque fraction (20 µl) est analysée par SDS-PAGE 12% (révélation : bleu de Coomassie) et Western blot.
   Les résultats sont représentés dans la figure 4A. Légende de la figure 4A :
   - = pas de sonication
   + = sonication
   S = protéines solubles
   I = protéines de membrane

   Les analyses par SDS-PAGE et Western blot des fractions d'enveloppe traitées montrent que la protéine IE41 n'est pas solubilisée après sonication des vésicules d'enveloppe. Au contraire, les protéines majeures solubles du stroma (RbcL) qui sont séquestrées dans les vésicules d'enveloppe, et qui sont connues pour contaminer les fractions d'enveloppe, sont solubilisées par ce traitement. Ceci montre que la protéine IE41 n'est ni une protéine soluble de l'espace intermembranaire, ni une protéine soluble du stroma contaminant la fraction d'enveloppe purifiée.
2) La protéine IE41 peut être liée à la membrane interne :
   - par ancrage à la bicouche lipidique ou insertion partielle dans celle-ci ; dans ce cas, seule l'utilisation de détergent peut permettre la solubilisation de la protéine IE41 ;
   - par des interactions électrostatiques avec une ou plusieurs protéines membranaires ou la surface polaire de la bicouche lipidique dans ce cas, ces interactions peuvent être rompues, et la protéine IE41 solubilisée, par un traitement alcalin ou par de fortes concentrations salines.

Pour déterminer le type d'interactions impliquées dans la liaison de IE41 avec la membrane interne, les expérimentations suivantes ont été effectuées :
a) solubilisation par le Triton X-100 :
   Des vésicules d'enveloppe (0,8 mg) sont diluées dans 1 ml de 50 mM MOPS, pH 7,8 contenant 0,05, 0,1 ou 0, 2% (v/v) de Triton X-100. Après incubation pendant 30 mn à 4°C, le mélange est centrifugé pour séparer les protéines solubles et membranaires. Toutes les fractions (20 µl) sont analysées par SDS-PAGE 12% (révélation : bleu de Coomassie) et Western blot.
   Les résultats sont illustrés par la figure 4C. Légende de la figure 4C :
   Mix = vésicules d'enveloppe purifiées
   M = protéines membranaires d'enveloppe
   S = protéines solubles d'enveloppe

   Ces résultats montrent que la protéine IE41 peut être totalement solubilisée par des concentrations de Triton X-100 (0,2%), beaucoup plus faibles que celles (de l'ordre de 2%) qui sont nécessaires pour solubiliser les protéines intrinsèques.
b) solubilisation par traitement alcalin ou par traitement salin.
   Des vésicules d'enveloppe purifiées (500 µg) sont incubées 30 mn à 4°C dans différents milieux (500 µl) :
   1) 10 mM MOPS, pH 7,8 + 0,5 M NaCl ;
   2) 10 mM MOPS, pH 7,8 + 0,5 M KI ;
   3) 0,1 M Na₂CO₃, pH 11 ;
   4) 0,1 N NaOH,
      puis soniquées et centrifugées comme décrit ci-dessus afin de séparer les protéines solubles et membranaires.

Toutes les fractions sont analysées (20 µl) par SDS-PAGE 12% (révélation : bleu de Coomassie) et Western blot (détection avec les anticorps polyclonaux dirigés contre la protéine IE41 d'*Arabidopsis* (dilution 1/5 000) ou dirigés contre la protéine IE18 (dilution 1/5 000).

Les résultats sont représentés dans la figure 4B
Légende de la figure 4B :
+ = sonication (10 sec)
NaCl 0,5 M = traitement 1
KI 0,5 M = traitement 2
0,1 M Na₂CO₃, pH 11 = traitement 3
0,1 N NaOH = traitement 4
S = fraction de protéines solubles
I = fraction de protéines insolubles

Ces résultats montrent que la protéine IE41 est au moins en partie solubilisée par les traitements salins (KI, NaCl) ou alcalins modérés (Na₂CO₃), qui sont sans effet sur la protéine intrinsèque IE18, cette dernière ne pouvant être solubilisée que par un traitement alcalin fort (NaOH).

L'ensemble des résultats présentés dans les figures 4A, 4B et 4C indiquent que la protéine IE41 est une protéine extrinsèque dont la liaison à la membrane interne de l'enveloppe chloroplastique implique des interactions électrostatiques.

### EXEMPLE 3 : PURIFICATION ET CARACTERISATION DE LA PROTEINE IE41 D'EPINARD

De façon surprenante, la protéine IE41 purifiée à partir de chloroplastes d'épinard et la protéine recombinante d'*Arabidopsis* présentent une taille similaire en SDS-PAGE et Western blot, ce qui évoque la possibilité que IE41 puisse être adressée à la membrane interne de l'enveloppe sans nécessiter le clivage d'une séquence d'import N-terminale.

Pour tester cette hypothèse, la protéine IE41 présente dans l'enveloppe des chloroplastes d'épinards a été purifiée afin de la séquencer et de comparer sa séquence à celle de l'ADNc correspondant.

### Immunopurification de la protéine IE41 d'épinard

Des protéines d'enveloppe chloroplastique (1 mg) sont solubilisées dans 1 ml de tampon Tris/HCl 50 mM, NaCl 150 mM, CHAPS 6 mM et centrifugées (20 mn, 72 000 g, Beckman L2 65B, rotor SW28). Les protéines solubles sont incubées pendant 1 h à 4°C avec 33 µl de sérum polyclonal dirigé contre la protéine recombinante IE41 d'*Arabidopsis.* On ajoute alors 50 µg d'agarose-protéine A (BOEHRINGER) et le mélange est incubé 3 h à 4°C. Après 3 lavages successifs par centrifugation (EPPENDORF 5415D, 12 000 rpm, 20 mn, 4°C) et remise en suspension du culot dans 1 ml de tampon de solubilisation (20 mM MOPS, 150 mM NaCl, 6 mM CHAPS, pH 7,8), on ajoute un excès (50 µg) de protéine recombinante (His-tag)-IE41 dans 200 µl de tampon de solubilisation. Le mélange est incubé pendant 1 h à 4°C, et centrifugé pendant 20 mn à 12 000 rpm (EPPENDORF 5415D). Le surnageant est incubé pendant 1 h avec de la résine Ni-NTA (QIAGEN), précédemment équilibrée dans le tampon de solubilisation, afin d'éliminer la majeure partie de la protéine recombinante (His-tag)-IE41.

Chaque fraction (20 µl) est analysée par SDS-PAGE 12% (révélation au nitrate d'argent) et Western blot (en utilisant les anticorps polyclonaux de lapin anti-IE41 décrits à l'Exemple 1).

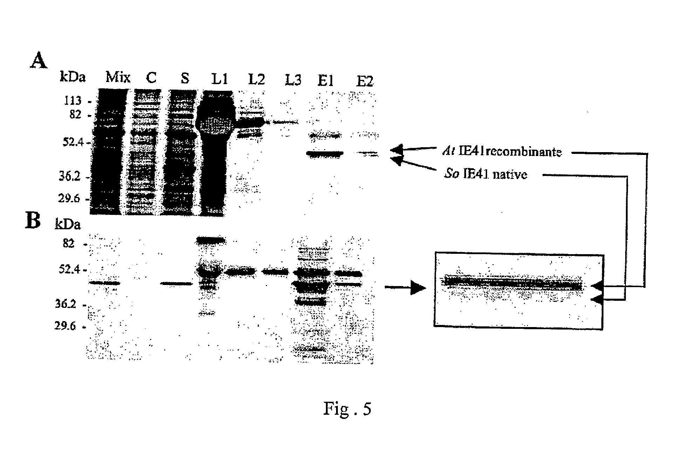

Les résultats sont représentés dans la figure 5. Légende de la figure 5 :
A : analyse par SDS-PAGE ;
B : Western blot ;
Mix = protéines d'enveloppe solubilisées ;
C = protéines insolubles ;
S = protéine solubles ;
L1, L2, L3 = fractions récupérées au cours des 3 lavages successifs ;
E1 = fraction éliminée par incubation avec la résine Ni-NTA;
E2 = protéine IE41 d'épinard purifiée (So) + (His-tag)-IE41.

La fraction E2 comprenant la protéine IE41 naturelle d'épinard purifiée (So) demeure contaminée par la protéine (His-tag)-IE41 recombinante d'*Arabidopsis.*

La différence de taille entre ces deux protéines correspond à l'extension polyhistidine (His-tag) présente à l'extrémité N-terminale de la protéine recombinante.

### EXEMPLE 4 : OBTENTION DE L'ADNc CODANT POUR LA PROTEINE IE41 D'EPINARD.

Le séquençage partiel de la protéine IE41 d'épinard éluée à partir du gel d'électrophorèse a permis d'obtenir 9 séquences peptidiques différentes. Ces séquences ont été utilisées pour définir des amorces dégénérées qui ont permis d'isoler l'ADNc codant pour la protéine IE41.

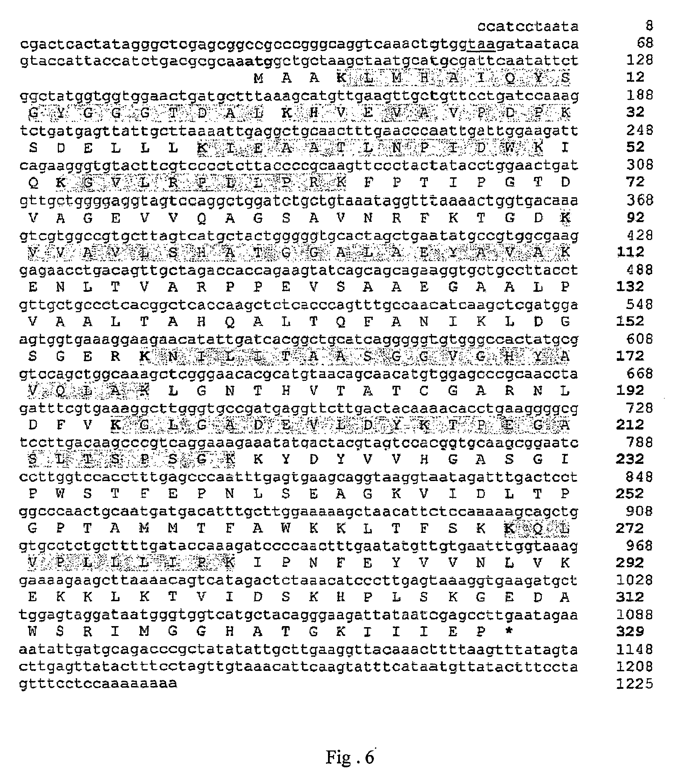

La séquence complète de cet ADNc (SEQ ID NO: 2), ainsi que la séquence polypeptidique déduite (SEQ ID NO:3) sont représentées sur la figure 6. Le codon ATG d'initiation de la traduction est indiqué en caractères gras, et le codon stop TAA est souligné. Les 9 séquences peptidiques obtenues par séquençage direct de la protéine IE41 d'épinard sont surlignées en gris. La correspondance entre les peptides obtenus avec la séquence déduite de l'ADNc de la protéine IE41 d'épinard, en particulier au niveau de la région N-terminale, ainsi que la présence d'un codon stop en aval de la méthionine initiatrice et dans le même cadre de lecture, démontrent que l'ADNc prédit est complet et que cette protéine ne subit pas de maturation post-traductionnelle lors de son adressage à la membrane interne de l'enveloppe du chloroplaste

La protéine IE41 d'épinard présente 75,1% d'identité et 88,8% de similarité avec la protéine IE41 d'*Arabidopsis.* Cette forte similarité, et le fait qu'Arabidopsis contient seulement un gène *ie41* par génome haploïde, permettent de conclure que ces protéines sont codées par des gènes d'*Arabidopsis* et d'épinard orthologues.

Les protéines IE41 d'*Arabidopsis* et d'épinard ont été alignées avec des protéines homologues de bactérie, de levure, et d'animaux.

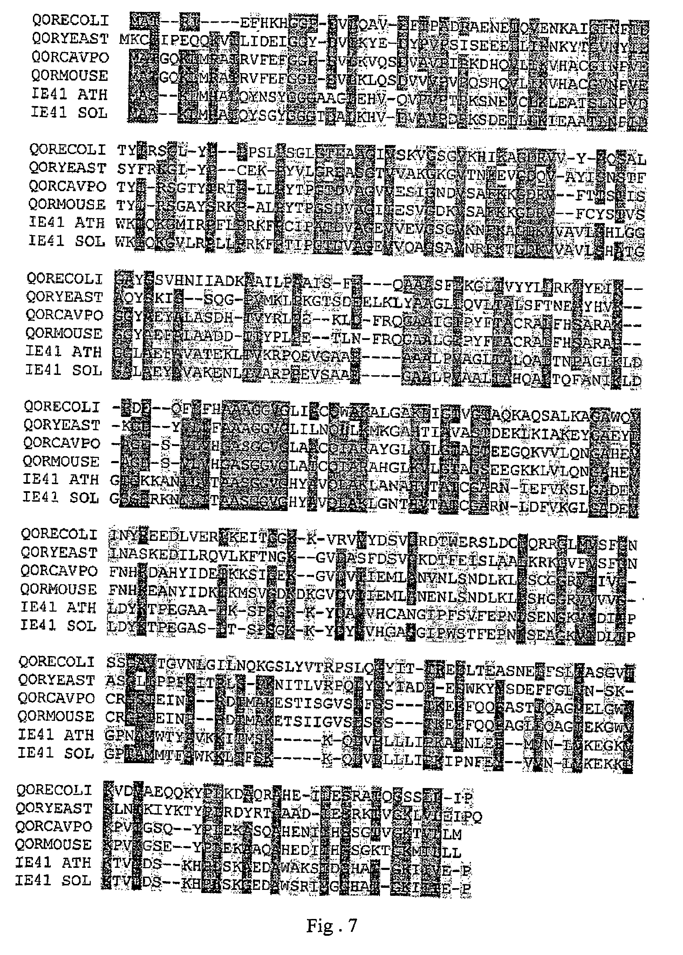

Les résultats sont présentés sur la figure 7.
*Arabidopsis thaliana* : IE41 ATH (SEQ ID NO : 1),
Epinard : IE41 SOL (SEQ ID NO : 3) ;
protéines homologues :
*Esherichia coli* : QORECOLI (SEQ ID NO : 6),
*Saccharomyces cerevisiae* : QORYEAST (SEQ ID NO : 7),
*Cavia Porcellus* : QORCAVPO (SEQ ID NO : 8),
souris : QORMOUSE (SEQ ID NO : 9).
Les résidus conservés dans les 6 séquences peptidiques sont surlignés en gris foncé. Les résidus conservés dans la séquence de IE41 et dans au moins une autre séquence de protéine homologue sont surlignés en gris clair. Les similarités entre résidus se basent sur les groupes suivants : ASPTG, ILMV, KRH, NQ, DE, YWF et C.

Les recherches d'homologies indiquent que la protéine IE41 appartient à la superfamille des deshydrogénases, et plus particulièrement au groupe des quinones oxydo-réductases de type ξ-crystalline (JÖRNVALL et al., FEBS 3, 240-244, 1993). De plus, la comparaison de séquence entre les protéines IE41 et les autres protéines de la même famille, révèle que les 50 premiers résidus dans la région N-terminale de ces protéines sont très conservés entre bactéries, végétaux, et animaux. Cette observation suggère que cette région N-terminale des protéines IE41 de végétaux ne serait pas impliquée dans l'adressage dans le chloroplaste, et serait conservée plus probablement du fait de la pression de sélection exercée au cours de l'évolution sur le domaine catalytique de la protéine.

### EXEMPLE 5 : ANALYSE DE L'ADRESSAGE PLASTIDIAL DE LA PROTEINE

### IE41 D'ARABIDOPSIS DANS DES CELLULES D'ARABIDOPSIS ET DE TABAC

Pour définir le domaine essentiel à l'import de la protéine IE41, différentes constructions codant pour des formes tronquées de cette protéine fusionnées à la GFP sont exprimées dans les cellules *d'Arabidopsis* et de tabac.

### Construction des vecteurs d'expression

Le plasmide [35Ω-sGFP(S65T) utilisé pour ces constructions, qui comprend la séquence codant pour la GFP sous contrôle du promoteur 35S, ainsi que le plasmide [35Ω-TP-sGFP(S65T)], qui comprend la séquence codant pour le peptide d'adressage (TP) de la petite sous-unité de la ribulose-1,5-bisphosphate carboxylase, fusionnée à la séquence codant pour la GFP ont été décrits précédemment par CHIU et al. (Curr. Biol., 6, 325-330, 1996).

La séquence codant pour la protéine IE41 d'*Arabidopsis* est amplifiée par PCR en utilisant les deux amorces suivantes :
*XhoI*-*N-ter* CCTCTCGAGATGGCTGGAAAACTCATGCAC (SEQ ID NO : 12),
   et
*NcoI-C-ter* CAACCCATGGATGGCTCGACAATGATCTTC (SEQ ID NO : 13), qui introduisent respectivement un site *XhoI* et un site *NcoI* (soulignés).

Le produit de PCR est cloné à bouts francs dans le vecteur PBLUESCRIPT KS (STRATAGENE). Le fragment *XhoI-NcoI* clivé du plasmide ainsi obtenu est inséré dans le plasmide 35Ω-sGFP(S65T) préalablement digéré par *SalI-NcoI* afin de créer le vecteur 35Ω-IE41-sGFP (S65T), comprenant la région codante de la protéine IE41 d'*Arabidopsis* fusionnée à la GFP. Un protocole similaire est utilisé pour les autres constructions :
*La séquence codant pour la protéine IE41 *d'Arabidopsis* dépourvue des 31 premiers acides aminés est obtenue par amplification PCR en utilisant les deux amorces suivantes :
   *SalI-N-ter* CGGTTGTCGACATGAAGAGTAATGAGGTTTGCCTG (SEQ ID NO : 14)
   *NcoI-C-ter* CAACCCATGGATGGCTCGACAATGATCTTC (SEQ ID NO : 13).

   Le plasmide 35Ω-Δ (1-31) IE41-sGFP (S65T) est obtenu par insertion de cette séquence dans le plasmide 35Ω-sGFP(S65T).
*La séquence codant pour la protéine IE41 d'*Arabidopsis* dépourvue des 59 premiers acides aminés est amplifiée par PCR en utilisant les deux amorces suivantes :
   *SalI-N-ter* GAATGGTCGACATGTTTCTGCCCCGCAAGTTC (SEQ ID NO : 15), et
   *Ncol-C-ter* CAACCCATGGATGGCTCGACAATGATCTTC (SEQ ID NO : 13).

   Le plasmide 35Ω-Δ (1-59) IE41-sGFP (S65T) est obtenu par insertion de cette séquence dans le plasmide 35Ω-sGFP(S65T).
*La séquence codant pour la protéine IE41 d'*Arabidopsis* dépourvue des 99 premiers acides aminés est amplifiée par PCR en utilisant les deux amorces suivantes :
   *SalI-N-ter* GGTTGTCGACATGCTAGGTGGAGGTGGACTTG (SEQ ID NO : 16)
   *NcoI-C-ter* CAACCCATGGATGGCTCGACAATGATCTTC (SEQ ID NO : 13).

   Le plasmide 35Ω-Δ (1-99) IE41-sGFP(S65T) est obtenu par insertion de cette séquence dans le plasmide 35Ω-sGFP(S65T).
*La séquence codant pour les acides aminés 6-100 de la protéine IE41 d'*Arabidopsis* est amplifiée par PCR en utilisant les deux amorces suivantes :
   *XhoI-N-ter* CCTCTCGAGATGGCTGGAAAAACTCATGCAC (SEQ ID NO : 17)
   *NcoI-C-ter* ACCCATGGCTAGATGGCTAAGAACCGCTAC (SEQ ID NO : 18).

   L'amorce SEQ ID NO : 17 comprend un nucléotide supplémentaire par rapport à l'amorce SEQ ID NO : 15, ce qui crée un décalage de la phase de lecture dans le produit d'amplification, dont la traduction débute au niveau du codon ATG correspondant à la méthionine en position 6 de la protéine IE41.
   Le plasmide 35Ω-(6-100) IE41-sGFP(S65T) est obtenu par insertion de cette séquence dans le plasmide 35Ω-sGFP(S65T).
*La séquence codant pour les acides aminés 60-100 de la protéine IE41 d'*Arabidopsis* est amplifiée par PCR en utilisant les deux amorces suivantes :
   *SalI-N-ter* GAATGGTCGACATGTTTCTGCCCCGCAAGTTC (SEQ ID NO : 15)
   *NcoI-C-ter* ACCCATGGCTAGATGGCTAAGAACCGCTAC (SEQ ID NO : 18).
   Le plasmide 35Ω-(60-100)IE41-sGFP(S65T) est obtenu par insertion de cette séquence dans le plasmide 35Ω-sGFP(S65T).

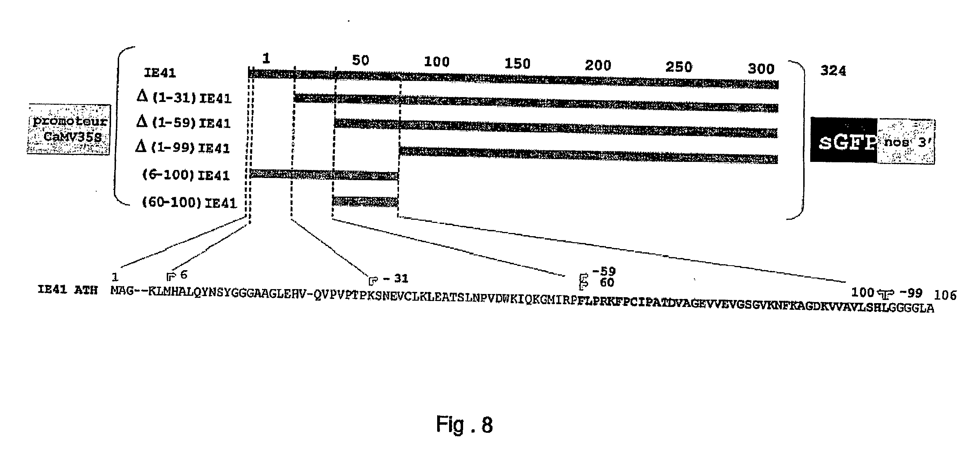

Ces différentes constructions sont représentées sur la figure 8.
Légende de la figure 8 :
IE41=plasmide 35Ω-IE41-sGFP(S65T)
Δ(1-31)IE41= plasmide 35Ω-Δ(1-31)IE41-sGFP(S65T)
Δ(1-59)IE41= plasmide 35Ω-Δ(1-59)IE41-sGFP(S65T) Δ(1-99)IE41=plasmide 35Ω-Δ(1-99)IE41-sGFP(S65T)
(6-100)IE41= plasmide 35Ω-(6-100)IE41-sGFP(S65T)
(60-100)IE41= plasmide 35Ω-(60-100)IE41-sGFP(S65T)

### Bombardement de cellules d'Arabidopsis et de tabac

Les cellules d'*Arabidopsis* sont cultivées à la lumière pendant 3 jours dans du milieu B5 de GAMBORG (SIGMA, pH 5,8) complémenté avec 1,5% sucrose et 1 µM ANA (acide naphtalène acétique). 15 ml de suspension cellulaire (correspondant à environ 0,5 g) sont transférés dans des boîtes de Pétri contenant le même milieu de croissance additionné de 0,8% bacto-agar, et incubés pendant 18-36 h à la lumière.

Les cellules BY2 de tabac sont cultivées pendant 5 jours à 27°C dans du milieu de MURASHIGE et SKOOG (milieu MS, DUCHEFA, pH 5,8) complémenté avec 3% sucrose, 0,2% KH₂PO₄, 0,2% myoinositol, 1 µM 2.4D (acide 2.4-dichlorophénoxyacétique) et 3 µM thiamine. 2,5 ml de suspension cellulaire (correspondant à environ 0,3 g) sont transférés dans des boîtes de Pétri contenant le même milieu de croissance additionné de 1% bacto agar et sont placés à 27°C pendant 18-24 h.

Les plasmides comprenant les constructions à tester utilisés pour le bombardement tissulaire sont préparés en utilisant le « QIAfilter Plasmid Midi Kit » (Qiagen, Allemagne).

Le plasmide [35Ω-sGFP(S65T)] (GFP) et le plasmide [35Ω-TP-sGFP(S65T)] (TP-GFP) sont respectivement utilisés à titre de témoin négatif et de témoin positif.

Les plasmides (1 µg) sont introduits dans les cellules en utilisant un canon à particules pneumatique (PDS-1000/He, BIORAD). Les conditions de bombardement sont les suivantes : pression d'hélium de 1 350 psi ; disques de rupture de 1 100 psi (BIORAD) ; distance de cible de 10 cm ; des microporteurs en or de 1 µm (BIORAD) sont utilisés. Après le bombardement, les cellules sont incubées sur ces mêmes boîtes de Petri pendant 18-36 h (à la lumière pour les cellules d'*Arabidopsis*), puis transférées sur des lames en verre avant la microscopie de fluorescence.

### Microscopie de fluorescence

La localisation de la GFP et des peptides de fusion à la GFP est analysée dans les cellules transformées par microscopie de fluorescence en utilisant un microscope à fluorescence ZEISS AXIOPLAN 2, et une caméra CCD digitale (HAMAMATSU). Les jeux de filtres utilisés sont : jeu de filtre Zeiss 13, 488013-0000 (excitateur BP 470/20, diviseur de faisceau FT 493, émetteur BP 505-530), et jeu de filtre Zeiss 15, 488015-0000 (excitateur BP 546/12, diviseur de faisceau FT 580, émetteur LP 590) pour la GFP et l'auto-fluorescence des chlorophylles, respectivement.

Dans ces conditions, la présence de la chlorophylle (spécifiquement localisée au niveau des chloroplastes) et la localisation de la GFP dans la cellule sont visualisées par une fluorescence intense.

Dans les cellules d'*Arabidopsis* transformées avec les constructions GFP, Δ(1-99)IE41, et (60-100)IE41, la fluorescence de la GFP apparaît diffuse, et localisée au niveau du cytosol et du noyau; aucune co-localisation avec la chlorophylle n'est observée.

Dans les cellules d'*Arabidopsis,* transformées avec les constructions IE41, Δ(1-31)IE41, Δ(1-59)IE41, (6-100)IE41, ainsi qu'avec le témoin positif de localisation TP-GFP, on observe au contraire une co-localisation, au niveau des chloroplastes, entre la fluorescence de la GFP et l'autofluorescence de la chlorophylle.

Les résultats sont similaires dans les cellules non chlorophylliennes BY2 de tabac : les marquages fluorescents observés avec les constructions IE41, Δ(1-59)IE41 et (6-100)IE41, et avec le témoin positif de localisation TP-GFP, correspondent à une localisation plastidiale ; en revanche, les marquages fluorescents observés avec les constructions GFP, Δ(1-99)IE41, et (60-100)IE41 correspondent à une localisation cytosolique et nucléaire.

Ces expériences montrent que l'adressage est également efficace dans les plastes non chlorophylliens.

L'ensemble des résultats ci-dessus montre que :
- la protéine IE41 complète fusionnée à la GFP est adressée dans le chloroplaste ;
- les 59 résidus localisés en N-terminal ne sont pas essentiels à l'import ;
- les 99 résidus localisés en N-terminal contiennent une région essentielle à l'import ;
- une séquence de 94 résidus, correspondant aux acides aminés N-terminaux 6-100 est suffisante pour catalyser l'import ; les 223 résidus (101-323) C-terminaux ne sont donc pas essentiels à l'import.

La séquence interne de 40 acides aminés, allant des résidus 60-100, correspond au domaine essentiel à l'import. Toutefois, ce domaine qui doit être présent pour diriger la protéine vers les plastes, n'est pas suffisant pour un bon adressage.

### EXEMPLE 6 : ANALYSE IN PLANTA DE L'ADRESSAGE PLASTIDIAL DE LA PROTEINE IE41

Les plasmides 35Ω-IE41-sGFP(S65T), 35Ω-Δ(1-31)IE41-sGFP(S65T) 35Ω-Δ(1-59) IE41-sGFP(S65T) 35Ω-Δ(1-99)IE41-sGFP(S65T), 35Ω-(6-100)IE41-sGFP(S65T), et 35Ω-(60-100)IE41-sGFP(S65T), ainsi que les plasmides témoins 35Ω-sGFP(S65T) et 35Ω-TP-sGFP(S65T), ont été digérés par EcoRI/HindIII pour récupérer les cassettes d'expression.

Celles-ci ont été insérées dans le plasmide binaire pEL103 (dérivé du plasmide pBI121 (AF485783), contenant un gène de résistance à la kanamycine), et le plasmide résultant a été utilisé pour transformer la souche C58 d'*Agrobacterium tumefaciens* par électroporation. Les bactéries transformées ont été utilisées pour transformer des plants d'*Arabidopsis* WS par la technique de « trempage floral » (The Plant Journal 1998; 16: 735-743). Les plantes transgéniques sont sélectionnées sur la base de leur résistance à la kanamycine.

Afin d'analyser l'expression des protéines de fusion dans les plantes transgéniques, les protéines totales sont extraites à partir de 10 mg de feuilles de chacune des plantes testées, et solubilisées dans le tampon suivant : pyrophosphate de tétrasodium (13,4 g/l), Tris-HCl pH 6,8 (50 mM), SDS (1 %).

L'extrait protéique est analysé par SDS-PAGE (12 % acrylamide), et par transfert de Western à l'aide d'un anticorps anti-GFP (anticorps 2A5 (Euromedex) au 1/4000^{e} dans TBST/lait 5 % ; anticorps secondaire: IgG anti-souris conjugué à la phosphatase alcaline (Promega) dilué au 1/10000^{e} dans TBS-Triton), ou des anticorps polyclonaux de lapin anti-IE41 décrits à l'Exemple 1 (au 1/5000^{e} dans TBS-Triton/lait 5% ; Anticorps secondaire IgG anti-lapin conjugué à la phosphatase alcaline (Promega) dilué au 1/10000^{e} dans du tampon TBS-Triton.

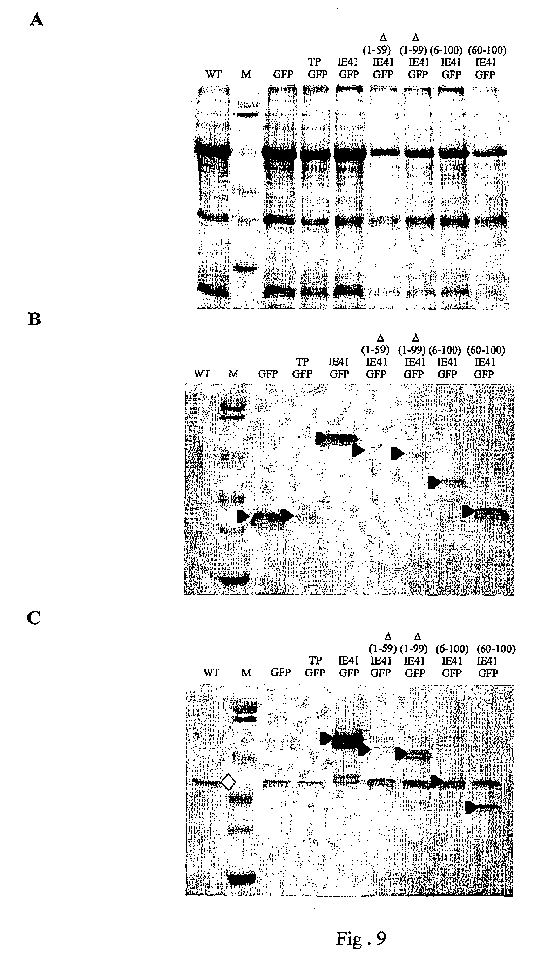

Les résultats de ces analyses sont illustrés par la Figure 9 ;
Légende de la Figure 9 :
- A : analyse par SDS-PAGE ;
- B : Transfert de Western avec l'anticorps anti-GFP : les flèches noires indiquent la présence de la protéine GFP dans les fusions exprimées chez *Arabidopsis ;*
- C : Transfert de Western avec un anticorps anti-IE41 : les flèches noires indiquent la présence de la protéine IE41 dans les fusions exprimées chez *Arabidopsis* ; le losange blanc indique la position de la protéine IE41 naturelle présente dans tous les extraits ;
- WT= plante non transformée ;
- M= marqueurs de poids moléculaire ;
- GFP= plasmide 35Ω-sGFP(S65T) ;
- TP GFP= plasmide 35Ω-TP-sGFP(S65T) ;
- IE41 GFP= plasmide 35Ω-IE41-sGFP(S65T) ;
- Δ(1-59)IE41 GFP= plasmide 35Ω-Δ(1-59)IE41-sGFP(S65T) ;
- Δ(1-99)IE41 GFP= plasmide 35Ω-Δ(1-99)IE41-sGFP(S65T) ;
- (6-100)IE41 GFP= plasmide 35Ω-(6-100)IE41-sGFP(S65T);
- (60-100)IE41 GFP= plasmide 35Ω-(60-100)IE41-sGFP(S65T).

Ces résultats montrent que les protéines de fusion sont exprimées dans toutes les plantes transformées.

La localisation subcellulaire des protéines exprimées par les différentes constructions a été visualisée en microscopie à fluorescence, comme décrit à l'Exemple 5 ci-dessus.

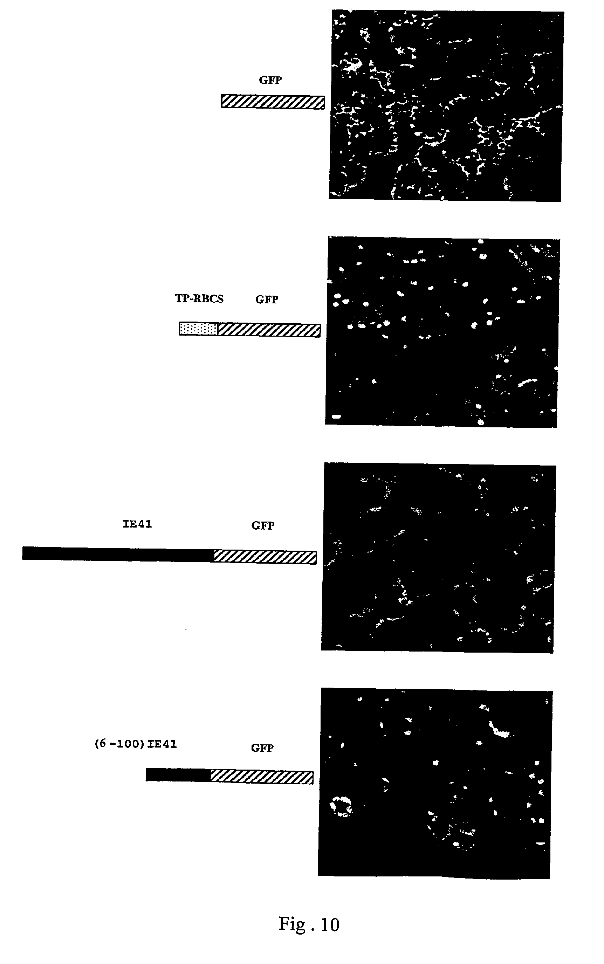

Les résultats sont illustrés par la Figure 10 ; Légende de la Figure 10 :
- GFP= plasmide 35Ω-sGFP (S65T) ;
- TP-RBCS GFP= plasmide 35Ω-TP-sGFP(S65T) ;
- IE41 GFP= plasmide 35Ω-IE41-sGFP(S65T) ;
- (6-100)IE41 GFP= plasmide 35Ω-(6-100)IE41-sGFP(S65T);

Il apparaît que, dans les conditions d'expression mises en oeuvre ci-dessus, c'est la région N-terminale de la protéine IE41 (résidus 6 à 100) qui confère la plus grande spécificité d'adressage vers le plaste. En effet la construction (6-100)IE41-GFP qui n'exprime que cette région, permet l'adressage systématique de la totalité de la fluorescence vers les plastes. Au contraire, la protéine IE41 complète (construction IE41-GFP) induit un adressage plastidial moins spécifique. Dans ces conditions, la protéine IE41 semble aussi adressée vers d'autres compartiments intracellulaires.

## Revendications

1. Polypeptide d'adressage intraplastidial **caractérisé en ce qu'**il est constitué par :
- un domaine A constitué par un polypeptide présentant au moins 60% d'identité, ou au moins 65% de similarité avec l'un des polypeptides SEQ ID NO: 4 ou SEQ ID NO: 5 ;
et au moins un domaine choisi parmi :
- un domaine B situé à l'extrémité N-terminale du domaine A, et constitué par un fragment de l'un des polypeptides SEQ ID NO: 1 ou SEQ ID NO: 3 comprenant au moins les acides aminés 49 à 59 dudit polypeptide, ou bien par un polypeptide présentant au moins 60% d'identité, ou au moins 65% de similarité, avec ledit fragment ;
- un domaine C situé à l'extrémité C-terminale du domaine A, et constitué par un fragment de l'un des polypeptides SEQ ID NO: 1 ou SEQ ID NO: 3 comprenant au moins les acides aminés 101 à 111 et au plus les acides aminés 101 à 151 dudit polypeptide, ou bien par un polypeptide présentant au moins 60% d'identité, ou au moins 65% de similarité avec ledit fragment.

2. Polypeptide selon la revendication 1, **caractérisé en ce que** le domaine B est constitué par un fragment comprenant au moins les acides aminés 39 à 59 des polypeptides SEQ ID NO :1 ou SEQ ID NO :3, ou bien par un polypeptide présentant au moins 60% d'identité, ou au moins 65% de similarité, avec ledit fragment.

3. Polypeptide selon l'une quelconque des revendications 1 ou 2, **caractérisé en ce que** le domaine C est constitué par un fragment comprenant au moins les acides aminés 101 à 121 des polypeptides SEQ ID NO :1 ou SEQ ID NO :3, ou bien par un polypeptide présentant au moins 60% d'identité, ou au moins 65% de similarité avec ledit fragment.

4. Polypeptide chimérique, comprenant un polypeptide d'adressage intraplastidial selon l'une quelconque des revendications 1 à 3 fusionné avec une protéine hétérologue.

5. Polypeptide chimérique selon la revendication 4, **caractérisé en ce que** le polypeptide d'adressage intraplastidial est placé à l'extrémité N-terminale de la protéine hétérologue.

6. Utilisation d'un polypeptide d'adressage intraplastidial selon la revendication 1 pour l'importation d'une protéine d'intérêt dans des plastes.

7. Utilisation selon la revendication 6, **caractérisée en ce que** ledit polypeptide d'adressage intraplastidial est utilisé pour l'importation de ladite protéine d'intérêt dans des chloroplastes.

8. Procédé pour importer une protéine d'intérêt dans des plastes **caractérisé en ce qu'**il comprend l'expression, dans une cellule végétale contenant lesdits plastes, d'un polypeptide chimérique résultant de la fusion d'un polypeptide d'adressage intraplastidial selon la revendication 1 avec ladite protéine d'intérêt.

9. Polynucléotide codant pour un polypeptide selon une quelconque des revendications 1 à 5.

10. Cassette d'expression comprenant un polynucléotide selon la revendication 9 placé sous contrôle de séquences de régulation de la transcription.

11. Vecteur recombinant résultant de l'insertion d'un polynucléotide selon la revendication 9 ou d'une cassette d'expression selon la revendication 10, dans un vecteur-hôte.

12. Plante transgénique transformée par, et comprenant, un polynucléotide selon la revendication 9 ou une cassette d'expression selon la revendication 10.

## Claims

1. Intraplastidial targeting polypeptide, **characterised in that** it is composed of:
- a domain A composed of a polypeptide having at least 60% identity, or at least 65% similarity, with one of the polypeptides SEQ ID NO: 4 or SEQ ID NO: 5;
and at least one domain selected from:
- a domain B situated at the N-terminal end of domain A and composed of a fragment of one of the polypeptides SEQ ID NO: 1 or SEQ ID NO: 3 comprising at least amino acids 49 to 59 of said polypeptide, or alternatively of a polypeptide having at least 60% identity, or at least 65% similarity, with said fragment;
- a domain C situated at the C-terminal end of domain A and composed of a fragment of one of the polypeptides SEQ ID NO: 1 or SEQ ID NO: 3 comprising at least amino acids 101 to 111 and at most amino acids 101 to 151 of said polypeptide, or alternatively of a polypeptide having at least 60% identity, or at least 65% similarity, with said fragment.

2. Polypeptide according to claim 1, **characterised in that** domain B is composed of a fragment comprising at least amino acids 39 to 59 of polypeptides SEQ ID NO: 1 or SEQ ID NO: 3, or alternatively of a polypeptide having at least 60% identity, or at least 65% similarity, with said fragment.

3. Polypeptide according to either claim 1 or claim 2, **characterised in that** domain C is composed of a fragment comprising at least amino acids 101 to 121 of the polypeptides SEQ ID NO: 1 or SEQ ID NO: 3, or alternatively of a polypeptide having at least 60% identity, or at least 65% similarity, with said fragment.

4. Chimeric polypeptide, comprising an intraplastidial targeting polypeptide according to any one of claims 1 to 3 fused with a heterologous protein.

5. Chimeric polypeptide according to claim 4, **characterised in that** the intraplastidial targeting polypeptide is placed at the N-terminal end of the heterologous protein.

6. Use of an intraplastidial targeting polypeptide according to claim 1 for importing a protein of interest into plastids.

7. Use according to claim 6, **characterised in that** said intraplastidial targeting polypeptide is used for importing said protein of interest into chloroplasts.

8. Method for importing a protein of interest into plastids, **characterised in that** it comprises the expression, in a plant cell containing said plastids, of a chimeric polypeptide resulting from the fusion of an intraplastidial targeting polypeptide according to claim 1 with said protein of interest.

9. Polynucleotide coding for a polypeptide according to any one of claims 1 to 5.

10. Expression cassette comprising a polynucleotide according to claim 9 placed sunder the control of sequences for regulating transcription.

11. Recombinant vector resulting from the insertion of a polynucleotide according to claim 9 or of an expression cassette according to claim 10 into a host vector.

12. Transgenic plant transformed by, and comprising, a polynucleotide according to claim 9 or an expression cassette according to claim 10.

## Patentansprüche

1. Intraplastid-Targeting-Polypeptid, **dadurch gekennzeichnet, dass** es besteht aus:
- einer Domäne A, die aus einem Polypeptid besteht, welches mindestens 60% Identität oder mindestens 65% Ähnlichkeit mit einem der Polypeptide SEQ-ID Nr. 4 oder SEQ-ID Nr. 5 aufweist;
und zumindest einer Domäne, die ausgewählt ist aus:
- einer Domäne B, die sich am N-terminalen Ende der Domäne A befindet und aus einem Fragment eines der Polypeptide SEQ-ID Nr. 1 oder SEQ-ID Nr. 3 besteht, welches zumindest die Aminosäuren 49 bis 59 dieses Polypeptids aufweist, oder aus einem Polypeptid besteht, welches mindestens 60% Identität oder mindestens 65% Ähnlichkeit mit diesem Fragment aufweist;
- einer Domäne C, die sich am C-terminalen Ende der Domäne A befindet und aus einem Fragment eines der Polypeptide SEQ-ID Nr. 1 oder SEQ-ID Nr. 3 besteht, welches zumindest die Aminosäuren 101 bis 111 oder höchstenfalls die Aminosäuren 101 bis 151 dieses Polypeptids aufweist, oder aus einem Polypeptid besteht, welches mindestens 60% Identität oder mindestens 65% Ähnlichkeit mit diesem Fragment aufweist.

2. Polypeptid gemäß Anspruch 1, **dadurch gekennzeichnet, dass** die Domäne B aus einem Fragment besteht, das zumindest die Aminosäuren 39 bis 59 der Polypeptide SEQ-ID Nr. 1 oder SEQ-ID Nr. 3 aufweist, oder aus einem Polypeptid besteht, das mindestens 60% Identität oder mindestens 65% Ähnlichkeit mit diesem Fragment aufweist.

3. Polypeptid gemäß einem der Ansprüche 1 oder 2, **dadurch gekennzeichnet, dass** die Domäne C aus einem Fragment besteht, das zumindest die Aminosäuren 101 bis 121 der Polypeptide SEQ-ID Nr. 1 oder SEQ-ID Nr. 3 aufweist, oder aus einem Polypeptid besteht, das mindestens 60% Identität oder mindestens 65% Ähnlichkeit mit diesem Fragment aufweist.

4. Chimärisches Polypeptid, das ein Intraplastid-Targeting-Polypeptid gemäß einem der Ansprüche 1 bis 3 aufweist, welches mit einem heterologen Protein fusioniert ist.

5. Chimärisches Polypeptide gemäß Anspruch 4, **dadurch gekennzeichnet, dass** sich das Intraplastid-Targeting-Polypeptid am N-terminalen Ende des heterologen Proteins befindet.

6. Verwendung eines Intraplastid-Targeting-Polypeptids gemäß Anspruch 1 zum Importieren eines interessierenden Proteins in Plasten.

7. Verwendung gemäß Anspruch 6, **dadurch gekennzeichnet, dass** das Intraplastid-Targeting-Polypeptid zum Importieren des interessierenden Proteins in Chloroplasten verwendet wird.

8. Verfahren zum Importieren eines interessierenden Proteins in Plasten, **dadurch gekennzeichnet, dass** es die Expression eines chimärischen Polypeptids, das aus der Fusion eines Intraplastid-Targeting-folypeptids gemäß Anspruch 1 mit dem interessierenden Protein resultiert, in einer diese Plasten enthaltenden pflanzlichen Zelle umfasst.

9. Polynucleotid, welches für ein Polypeptid gemäß einem der Ansprüche 1 bis 5 codiert.

10. Expressionskassette, die ein Polynucleotid gemäß Anspruch 9 aufweist, welches der Kontrolle von Sequenzen zum Regulieren der Transkription unterliegt.

11. Rekombinanter Vektor, der aus dem Einbau eines Polynucleotids gemäß Anspruch 9 oder einer Expressionskassette gemäß Anspruch 10 in einen Wirts-Vektor resultiert.

12. Transgene Pflanze, die durch ein Polynucleotid gemäß Anspruch 9 oder eine Expressionskassette gemäß Anspruch 10 transformiert ist und ein solches bzw. eine solche aufweist.
